# EUROPEAN PATENT APPLICATION

(11) **EP 1 882 746 A2**
(43) Date of publication of application: **30.01.2008**
(21) Application number: 07111614.9
(22) Date of filing: 12.07.2007
(51) Int. Cl.: C12Q 1/68

(54) **Method for detecting a methylation pattern**

(30) Priority: 27.07.2006 EP 06076487
(71) Applicant: Epigenomics AG, 10178 Berlin (DE)
(72) Inventor: Berlin, Kurt, 14532 Stahnsdorf (DE); Distler, Jürgen, 12163 Berlin (DE); Tetzner, Reimo, 10439 Berlin (DE)

(57) **Abstract**

The present invention relates to a method for detecting the presence of one or more methylation patterns. It comprises the conversion of nucleic acids and a catalytic nucleic acid activity. Here, the conversion of the nucleic acid is characterized such that the 5-methylcytosine remains unchanged while the unmethylated cytosine is converted into uracil or another base, which can be distinguished from cytosine in its base-pairing behavior.

<110> Epigenomics AG

<120> Method for detecting a Methylation Pattern

<160> 12

<210> 1
<211> 20
<212> DNA
<213> Homo Sapiens

<400> 1

gttttcgttg gagtttcgtc                                                    20

<210> 2
<211> 52
<212> DNA
<213> Homo Sapiens

<400> 2

ctctcctcat cgttgtagct agcctgggta ctcactaata acgaaaacta cg                52

<210> 3
<211> 32
<212> DNA
<213> Homo Sapiens

<400> 3

ctctcctcag ugggtactca ctaataacga aa                                      32

<210> 4
<211> 46
<212> DNA
<213> Homo Sapiens

<400> 4

gttttcgttg gagtttcgtc gtcgtagttt tcgttattag tgagta                       46

<210> 5
<211> 41
<212> DNA
<213> Homo Sapiens

<400> 5

ttattagtga gtacccaggc tagctacaac gatgaggaga g                            41

<210> 6
<211> 19
<212> DNA
<213> Homo Sapiens

<400> 6

gggaggggtt aggagtgta                                                     19

<210> 7
<211> 50
<212> DNA

<213> Homo Sapiens

<400> 7

cacaccactt cgttgtagct agcctgggaa aaaacctatt tccctattcc          50

<210> 8
<211> 30
<212> DNA
<213> Homo Sapiens

<400> 8

cacaccactg ugggaaaaaa cctatttccc          30

<210> 9
<211> 46
<212> DNA
<213> Homo Sapiens

<400> 9

gggaggggtt aggagtgtag ggagggaata gggaaatagg tttttt          46

<210> 10
<211> 44
<212> DNA
<213> Homo Sapiens

<400> 10

gggaaatagg tttttcccca ggctagctac aacgaagtgg tgtg          44

<210> 11
<211> 74
<212> DNA
<213> Homo Sapiens

<400> 11

gttttcgttg gagtttcgtc gtcgtagttt tcgttattag tgagtaccca ggctagctac          60
aacgatgagg agag          74

<210> 12
<211> 74
<212> DNA
<213> Homo Sapiens

<400> 12

gggaggggtt aggagtgtag ggagggaata gggaaatagg tttttcccca ggctacgtac          60
aacgaagtgg tgtg          74

## Description

The present invention relates to a process for detecting a methylation pattern in a nucleic acid.

In this application, various publications are being cited. The disclosure of the publications is herewith included in the description to describe the relevant state of the art more fully in this application.

### Background of the Invention

5-methylcytosine is the most covalently modified base in the DNA of eukaryotic cells. It plays an important biological role, for example, in transcription regulation, genetic imprinting, and in tumorigenesis (for an overview: Millar et al.: Five not four: History and significance of the fifth base. In: The Epigenome, S. Beck and A. Olek (eds.), Wiley-VCH Verlag Weinheim 2003, P. 3-20). The identification of 5-methylcytosine as an integral part of genetic information is, therefore, of great interest.

The detection of methylation is, however, difficult because cytosine and 5-methylcytosine exhibit identical base-pairing behavior. Many of the conventional detection methods, based on hybridization techniques, do not have the capacity to distinguish between cytosine and 5-methylcytosine. Furthermore, the methylation information during an amplification reaction, using the polymerase chain reaction (PCR), is completely lost.

The conventional methods for methylation analysis basically work according to two significantly different principles. In one case, methylation-specific restriction enzymes are used, and in another, selective chemical conversion takes place of unmethylated cytosine into uracil (so-called: bisulfite-treatment, see also: DE 101 54 317 A1; DE 100 29 915 A1). The enzymatic or chemically treated DNA can be then in most cases amplified and is analyzed using different methods (for an overview: WO 02/072880 P 1 ff).

According to the bisulfite method, cytosine bases are changed to uracil whereas 5-methylcytosine remains unchanged (Shapiro et al. (1970) Nature 227: 1047)). Uracil behaves with respect to base-pairing like thymine, and thus, forms a base-pair with adenine. Even after a bisulfite-treatment, 5-methylcytosine hybridizes, like before, with guanine.

As a result of this base conversion, it is then possible to distinguish between methylated and unmethylated cytosine bases.

Because the use of methylation-specific restriction enzyme requires the recognition of certain sequences, most of the detection methods for methylated DNA are based on bisulfite treatment, which is carried out prior to detection or amplification (for example: DE 100 29 915 A1, page 2, lines 35-46; see also WO 2004/067545). The term bisulfite-treatment comprises the treatment with a bisulfite-, disulfite-, or a hydrogensulfite-solution. It is known to the person skilled in the art that the term "bisulfite" is used synonymously with the term "hydrogensulfite".

In the state of the art, various laboratory protocols are known, which all comprise the following steps: genomic DNA is isolated, denatured, undergoes a conversion reaction for several hours using a concentrated bisulfite solution, is desulfonated, and desalted (i.e.: Frommer et al. (1992) A genomic sequencing protocol that yields a positive display of 5-methylcytosine residues in individual DNA strands. Proc Natl Acad Sci U. S. A.; 89(5): 1827-1831).

Of great interest are methods, which are able detect methylation sensitively and quantitatively. This is essential particularly with regard to diagnostic applications due to the important role cytosine-methylation has in cancer development. Of special significance are methods that allow for the detection of changing methylation patterns in body fluids, such as serum. As opposed to unstable RNA, DNA is often encountered in body fluids.

Due to the destructive pathological processes found in cancer, the DNA concentration in blood is elevated. A cancer diagnosis through a methylation analysis of body fluids carrying tumor DNA is thereby possible and has been described many times already (see: Palmisano et al.: Predicting lung cancer by detecting aberrant promoter methylation in sputum. Cancer Res. 2000 Nov 1;60(21): 5954-8).

However, there exists a problem: that is, in addition to the DNA with methylation patterns typical of a disease-state, one finds a large amount of DNA of the same sequence but with a different methylation pattern. The diagnostic methods must, therefore, have the ability to detect low amounts of DNA available with variable methylation patterns out of a preponderant background of DNA of the same sequence but normal methylation.

The common method for sensitive detection is carried out via a PCR-amplification. One method is the so-called methylation-sensitive PCR ("MSP"; Herman et al.: Methylation-specific PCR: a novel PCR assay for methylation status of CpG islands. Proc Natl Acad Sci U S A. 1996 Sep 3; 93(18): 9821-6). Primers are used for this that bind exclusively to Bisulfite-treated sequences, which were either methylated (or through an inverse approach, unmethylated) beforehand.

A method with similar sensitivity is the so-called "Heavy Methyl" method. Thereby, a specific amplification is obtained only of the original methylated (or unmethylated) DNA through the use of methylation-specific blocking oligomers (for an overview: WO 02/072880).

The MSP method as well as the Heavy Methyl method can be applied as quantifiable real-time variations. They make it possible to detect the methylation status of a few positions directly in the course of the PCR without the necessity for a subsequent analysis of the product ("MethyLight" - WO 00/70090; US 6,331,393).

An embodiment of the said real-time methods is the "Taqman" method. This utilizes probes, which carry a pair of fluorescence dye and a quencher. The probes hybridize in a sequence-specific manner to the amplified products and in the course of the subsequent amplification cycles, are degraded through the exonuclease activity of the polymerase. As a result of the separation of the quencher from the dye, a detectable fluorescent signal is produced (see Eads et al.: MethyLight: a high-throughput assay to measure DNA methylation. Nucleic Acids Res. 2000 Apr 15; 28(8): E32).

A further embodiment of the MethyLight method is the so-called Lightcycler method. In this case, two different probes are deployed that hybridize within the immediate vicinity of each other to the amplified product, and then through the Fluorescence-Resonance-Energy-Transfer (FRET), a detectable signal is produced.

An overview of real time PCR methods can be gathered form Kaltenboeck B, Wang C. Advances in real-time PCR: application to clinical laboratory diagnostics. Adv Clin Chem. 2005;40:219-59.

The applicability of these real-time methods for methylation analyses is, however, limited. This is true particularly in relation to specificity, sensitivity, and reaction speed. Due to the special biological and medical significance of cytosine methylation, there exists a strong need for the technical development of a high performance method for methylation analysis. In the following pages, such a method is described. Herein, a catalytically active nucleic acid, which is generated through amplification, is used to detect amplificates. According to the invention, the method allows for an effective and fast detection and affords a very sensitive and specific methylation analysis.

A method similar to the method of the present invention for the analysis of mutations has been published in US-Patent US 6,140,055. This method can be applied in a variety of embodiments. It is common to all variants that a catalytically active nuclei acid is produced during a PCR amplification reaction. The enzymatic activity is being used to detect the amplificate. In a preferred embodiment of the method, a primer comprises a reverse complementary sequence of the catalytically active nucleic acid. However, in contrast to conventional real-time-PCR methods that which are used for mutational analyses, this method possesses no advantages. For the person skilled in the art, the use of the mutational analysis method in methylation analysis is not obvious because in contrast to mutational analysis, an additional step is necessary for methylation analysis, in particular, the bisulfite-treatment of DNA. The transfer of the known mutational analysis method for use in methylation analyses cannot be easily performed, since bisulfite-treated DNA is different chemically and physically from genomic DNA in multiple ways; this includes, for example: the length, the complexity, base composition, and the structure:
As a result of the bisulfite conversion, the DNA becomes highly fragmented. The level of fragmentation is dependent on the reaction conditions; the longer the reaction, the higher the temperature, the greater the degradation rate is of the DNA. To achieve complete conversion, however, correspondingly long reaction times are necessary. The resulting bisulfite-treated DNA is a complex mixture of DNA of various lengths and incompletely converted fragments (for an overview: Grunau et al 2001: Bisulfite genomic sequencing: systematic investigation of critical experimental parameters. Nucleic Acids Res. 2001 Jul 1; 29(13): E65-5). Since the incompletely converted DNA may be a source for false-positive signals, it must be ensured that only converted DNA is detected.
The bisulfite-treated DNA contains different bases than genomic DNA. As a result of the bisulfite conversion, all the non-methylated cytosine bases become converted into uracil, which does not exist in genomic DNA. Genomic DNA is composed of four bases. Bisulfite-treated DNA, however, contains over large areas only three basses, since all the unmethylated cytosines have been converted, namely at least all those cytosines, which are found outside of the CpG sequence context. There are a multitude of enzymes that are able to distinguish between uracil and thymine.
Because of this 3-base alphabet, the bisulfite-treated DNA is less complex than genomic DNA. The appearance of repetitive sequences is more frequent. Therefore, the design of specific primers or probes for bisulfite-treated DNA is far more difficult than for genomic DNA.
Genomic DNA has a characteristic double-helix structure. The bisulfite-treated DNA exists, however, not in a double-stranded but in a single-stranded form. This stems from the fact that after the bisulfite conversion, two uncomplimentary DNA strands emerge that can no longer hybridize with one another (While C is converted to U on one strand, in the opposing strand, the complementary G remains unchanged. The G cannot form a bond with the U anymore). The single-stranded DNA is composed of, however, possibly short sections of double-stranded DNA, which is favored by the low complexity of bisulfite-treated DNA (see above).

Genomic DNA and bisulfite-converted DNA are different from one another chemically and physically in many different ways. Methods to analyze genomic DNA are, therefore, off hand not adaptable for the analysis of bisulfite-treated DNA.

Moreover, according to US 6,140,055, there are also biological reasons for why the mutational analysis method is off hand not transferable for analysis of methylation. One reason is that most mutations or single nucleotide polymorphisms (SNP's) appear as individual and isolated base changes. In order to detect this change, it is necessary to resolve the single base-pair mutation between the probe and the DNA.

In contrast, the situation in methylation analysis is different. The CpG positions appear often in CpG-rich regions (so-called CpG-islands). Here the neighboring CpG positions often show identical methylation status ("co-methylation"). Particularly, DNA, retrieved from cancer cells out of body fluids, show uniform methylation patterns for hundreds of base pairs. This co-methylation can be used for methylation analysis, since through a simultaneous analysis the specificity of the detection of neighboring cytosine positions can be enhanced.

Another important difference between a mutation and cytosine methylation consists of the fact that a mutation deals with a stable, irreversible change in the DNA. There are only two possibilities: mutated and not mutated. An exact quantification of a mutation is often unnecessary because the mutation appears in the DNA of all cells being sampled.

In contrast to that, cytosine methylation is a flexible change in the DNA. The DNA in different tissues is methylated differently. Tumors show different methylation levels in a variety of developmental phases or stages of aggressiveness (i.e.: Jeronimo et al, J Natl Cancer Inst 2001 Nov 21; 93(22): 1747-52). An exact quantification of methylation is, therefore, necessary for many important applications, such as: for the classification of tumors, for prognoses, and the prediction of patient response to certain medications.

Moreover, the analysis of cytosine methylation particularly from body fluids poses the following problem: besides the DNA with a specific methylation pattern from tumors being located in the body fluids, the body fluids also contain an abundance of DNA that originates from natural breakdown and ageing processes. The "not-pathological" DNA has the identical sequence as the tumor-DNA and differs from the other only through its methylation pattern. Diagnostic analysis methods must be specifically able to detect small amounts of tumor-DNA from this background. There is no comparable situation in SNP analysis.

The underlying problem of the present invention was to improve the detection of methylated and non-methylated cytosine bases in the DNA molecule, particularly in regard to specificity and sensitivity.

### Description

### Advantages

According to the invention, the method is surprisingly well suited for methylation analysis. This is founded on the fact that the probe, used for detection, does not bind to the bisulfite-treated DNA but to an amplified portion of it. Surprisingly, this produces an increase in probe specificity and sensitivity and also a general enhanced sensitivity of the analysis as compared to conventionally used methods for methylation analyses. In these methods, the probe hybridizes to the bisulfite-treated DNA or an amplified portion of it. According to the invention, the method clearly demonstrates an enhanced specificity and sensitivity as compared to the conventional methods used for methylation analysis.

The enhanced specificity and sensitivity of the probe according to the invention is founded on it comprising four bases. In comparison, probes from conventional methods for methylation analysis are comprised of basically only three bases. The fourth base in these probes can only appear in order to ascertain the methylation of a position. Based on the fact that all four bases can appear in the probe according to the invention, probes can be generated that can bind to the target-DNA at high temperatures. This is equivalent with higher sensitivity. In addition, the presence of four bases in the probe enables it to have enhanced specificity. This is because with four bases a more complex and therefore, a more unequivocal pattern can be generated than with three bases.

The methods used, thus far, for methylation analysis have been restricted to probes made up of basically three bases because they bind to bisulfite-treated DNA, also made up of only three bases. Unmethylated cytosine is converted through the bisulfite treatment into Uracil, which exhibits the same base-pairing behavior as thymine. It concerns both the cytosines outside of CpG positions and the unmethylated cytosines within the CpG position. Methylated cytosine continues to exist as cytosine after bisulfite treatment, however, it occurs comparatively infrequently. Amplified, bisulfite-treated DNA consists of mainly three bases: A, T, and G or in the opposite strand A, T, and C. The fourth base occurs only in the rare cases of methylation.

Moreover, the method according to the invention possesses an enhanced sensitivity in comparison to conventional methods used for methylation analysis because small fragments are also taken into consideration. Since the probes according to the invention bind outside of the primer binding sites of the amplified product, the distance between the primers can be kept small. This, however, is not possible in conventional methods. Here, the distance of the primer must be chosen in such a manner that the binding of one or two probe(s) and optionally of the blockers remains possible.

As has been mentioned above, two real-time methods have been predominantly used until now for methylation analysis. 1) In the so-called Taqman method, probes, which feature a pair of a fluorescent-dye and a quencher, are used. They bind to the DNA to be amplified and are degraded during the amplification cycles through the exonulcease activity of the polymerase. 2) In the Lightcycler method, two different kinds of probes are used that hybridize within the immediate vicinity of each other to the amplificate, and then through the Fluorescence Resonance Energy Transfer (FRET) generate a detectable signal.

On the basis of the following disadvantages, the utility of the Taqman method for methylation analysis is limited: as has been already exemplified, bisulfite-treated DNA has a reduced complexity. It is made up of essentially three base codes. Therefore, it is necessary that the probes have an average length of between 21 and 26 nucleotides in order to guarantee the specific binding of the probe to the bisulfite-treated DNA. CG probes used to detect methylated DNA as a rule are about 21 nucleotides long, while TG-probes used to detect unmethylated DNA comprise usually 26 nucleotides. Typically, each of the two primers comprises between 20 and 25 nucleotides. Based on these numbers, only DNA fragments from about 70 nucleotides long can theoretically be analyzed using these primers. In practice, however, this number is significantly higher, since, the two primers as well as the probe have to be chosen such that they are specific.

The Lightcycler method is even less applicable, since the specific hybridization of the two probes on the generated amplificate must be guaranteed. Theoretically, the lower limit of the fragments that can be examined is about 95 nucleotides. In practice, however, the number is considerably greater because of the reason given above. Therefore, the Lightcycler method cannot be used for small DNA fragments.

The possibility of the method according to the invention to include small DNA fragments, also comprising below a length of 70 nucleotides into the analysis, is especially advantageous. The method is additionally advantageous because, as a result of the bisulfite treatment, the DNA is fragmented. The fragmentation limits a methylation analysis particularly when the DNA is already highly fragmented. That is the case in tumor diagnosis from body fluids. Thus, tumor DNA is easily degraded by DNAses. Another example is DNA from paraffin-embedded formalin-fixed tissue. DNA becomes highly fragmented through formalin treatment. DNA from paraffin-embedded formalin-fixed tissue is particularly used to discover and identify new methylation specific markers. High performance methods must therefore be in the position to analyze also short, bisulfite-treated DNA segments.

The enhanced sensitivity and specificity of the method according the invention are surprising to the person skilled in the art because for mutational analysis it is unimportant whether a probe hybridizes within or outside of the amplified region. This is particularly the case with bisulfite-treatment because this is not necessary and does not take place. As a result, there is no reduction of complexity of the examined DNA or additional fragmentation of the DNA as in the case of methylation analysis.

The method according to the invention offers yet another advantage in that it can be performed both as a normal PCR as well as real-time PCR. Both variations allow quantification of the amplificate or the bisulfite-converted DNA to be amplified.

Furthermore, the method according to the invention has the advantage in that several different DNA molecules can be simultaneously analyzed and quantified. Here, it is possible to simultaneously analyze and quantify either one as well as multiple methylation positions at the same time.

The method according to the invention is therefore able to improve the detection of methylated and unmethylated cytosine bases in a DNA molecule as compared to the conventional methods, especially with regard to enhanced specificity and sensitivity.

### Aspects of the Invention

The method according to the invention is a method to detect the presence of one or more methylation patterns, comprising a) the conversion of nucleic acid, so that 5-methylcytosine remains unchanged, while unmethylated cytosine is converted into uracil or another base, which can be distinguished from cytosine in its base-pairing behavior, and b) a catalytic nucleic acid activity. The method is a method to detect the presence of one or more methylated or unmethylated cytosine bases. The cytosines, to be analyzed, can be either co-methylated or not. To the person skilled in the art, one or more cytosines that are co-methylated or not are known as a methylation pattern. The method according to the invention allows not only the analysis of a methylation pattern with regard to one DNA molecule but also from multiple molecules. The methylation pattern can be detected here as well as quantified. Consequently, the degree of methylation and, as the case may be, the fraction of molecules can be determined that exhibit a certain methylation pattern. This is, for example, indicated for the analysis of tissue samples, which contain not only tumor cells but also benign cells. The method according to the invention is able to detect and quantify a methylation pattern of tumor DNA from a mixture of DNA extracted from tumor and benign cells.

The method according to the invention is a method to detect the presence of a methylation pattern in a sample nucleic acid, comprising
a) the conversion of nucleic acid to be examined from a sample, so that 5-methylcytosine remains unchanged, while unmethylated cytosine is changed to uracil or another base, which can be distinguished from cytosine in its base-paring behavior,
b) bringing into contact the nucleic acid to be examined with a zymogen, encoding a nucleic acid catalytic activity, thereby constituting its reverse complementary sequence whereby a nucleic acid molecule is generated that comprises a catalytic nucleic acid activity, and
c) the detection of a catalytic nucleic acid activity through which the presence of a methylation pattern in the nucleic acid to be examined is determined.

In the first step of the method according to the invention, the nucleic acid to be examined is converted either chemically or with an enzyme so that the 5-methylcytosine remains unchanged while unmethylated cytosine is changed to uracil or another base, which can be distinguished from cytosine in its base-paring behavior.

In the following, the chemically or enzymatically treated nucleic acid to be examined will be described as "converted", "transformed", or "treated" nucleic acid.

The nucleic acid to be examined may come from a variety of sources depending on the diagnostic or scientific question. For diagnostic questions, tissue samples and body fluids may serve as the preferred source material. However, tissue samples are, for example, but not limited to biopsies or paraffin-embedded formalin-fixed biopsies. Body fluids, however, are, for example, but not limited to blood, serum or plasma.

It is also possible to use nucleic acid from sputum, stool, urine, spinal fluid, saliva, sperm, fluid from the pleural space, fluid from peritoneal space, cerebro-spinal fluid, swab from an epithelial surface, lymph fluid, meningial fluid, glandular fluids or any other known body fluids.

Preferentially, the nucleic acid will first be isolated from the biological sample. The extraction carried out by standard methods, which the person skilled in the art would know. Preferentially, the extraction carried out through the use of a kit known to the person skilled in the art. For example, but not limited to, the DNA extraction from plasma is performed using QlAamp UltraSens Virus Kit (Qiagen). For example, but not limited to, the RNA-extraction is performed from paraffin-embedded formalin-fixed biopsies using the QlAamp UltraSens Virus RNA Mini Kit (Qiagen). The isolated nucleic acid can then be fragmented, if necessary. This can be carried out using physical, chemical, or biological methods either before or after the bisulfite conversion. For example, but not limited to, the fragmentation of DNA carried out by using restriction enzymes. The reaction conditions and the question of suitable enzymes are known to the person skilled in the art may be deduced from the protocols provided by the manufacturer.

Subsequently, the DNA is converted chemic ally or enzymatically. Preferentially, the chemical conversion is carried out using bisulfite. The different variations in bisulfite conversion are known to the person skilled in the art (see: Frommer et al.: A genomic sequencing protocol that yields a positive display of 5-methylcytosine residues in individual DNA strands. Proc Natl Acad Sci U S A. 1992 Mar 1; 89(5): 1827-1831; Olek, A modified and improved method for bisulfite based cytosine methylation analysis. Nucleic Acids Res. 1996 Dec 15; 24(24): 5064-5066.; DE 100 29 915). Especially preferred is that the bisulfite conversion takes place in the presence of denaturing substances, such as, polyethylene glycol dialkyl ethers or dioxane, and a radical scavenger (compare: DE 100 29 915).

Similarly, the bisulfite conversion is carried out preferentially in the presence of scavengers like, for example, chromium derivatives, such as, 6-Hydroxy-2,5,7,8,-tetramethylchromane-2-carboxyl acid or 3,4,5,-trihydroxybenzoic acid, or its derivatives gallic acid (see: PCT/EP2004/011715).

The bisulfite conversion is carried out preferentially at a reaction temperature between 30 °C and 79 °C whereas for a short period of time, the temperature is raised to 85 °C (see: PCT/EP2004/011715). The bisulfite-converted DNA is preferentially purified before it is quantified. This can be carried out by state-of-the-methods like ultrafiltration, preferably by Microcon^{™} columns (Millipore^{™}). The purification will be carried out according to the protocols provided by the manufacturer (see: PCT/EP2004/011715).

Furthermore, it is preferable that the bisulfite-converted DNA is directly quantified without being previously desulfonated (see PCT/EP2005/011133).

In another preferred embodiment, the DNA is converted not chemically but enzymatically. This is possible through the use of cytidine deaminase, which can convert unmethylated cytidine faster than methylated cytidine. A corresponding enzyme is described in Bransteitter et al.: Activation-induced cytidine deaminase deaminates deoxycytidine on single-stranded DNA but requires the action of RNase. Proc Natl Acad Sci U S A. 2003 Apr 1; 100(7): 4102-4107; WO 2005/005660.

In the second step of the method according to the invention, the treated, converted nucleic acid to be examined will be brought into contact with a nucleic acid. It encodes a catalytic nucleic acid activity, that is, it comprises the reverse complementary sequence of the catalytic nucleic acid activity. This will be denoted in the following as a zymogen.

In the third step of the method according to the invention, the catalytic nucleic acid activity is detected. Thereby, the existence of a methylation pattern of the nucleic acid to be examined can be determined.
A preferred embodiment comprises additionally in the second step of the method to bring in contact with the nucleic acid to be examined an oligonucleotide, which comprises a region that also serves as a primer. This primer enables the amplification of the nucleic acid. The reaction conditions in the second step of the method are chosen so that it allows for the primer-initiated nucleic acid amplification as well as an activity of a catalytic activity of the nucleic acid.

The oligonucleotide (or the primer) and the zymogen would be oriented with one another such that in the presence of the converted nucleic acid to be examined, a nucleic acid would be generated. This nucleic acid would be characterized such that it comprises a region that contains either a sequence or a reverse complementary sequence of the nucleic acid to be examined. Additionally, it comprises a region that is the reverse complementary sequence of the zymogen, which thereby, is the catalytic nucleic acid activity.

The nucleic acid amplification is catalyzed preferably using a polymerase, especially preferred, a thermostable polymerase. It can be an advantage when the polymerase used does not posses an exonuclease activity.

A preferred embodiment of the method according to the invention is a method to detect the presence of a methylation pattern in the nucleic acid of a sample, comprising
a) the conversion of a nucleic acid of a sample such that 5-methylcytosine remains unchanged while unmethylated cytosine is converted into uracil or into another base, which can be distinguished from cytosine by its base-pairing behavior,
b) the subjecting of the nucleic acid to be examined to
   i) an oligonucleotide appropriate for the initiation of nucleic acid amplification, and
   ii) a zymogen, which encodes a catalytic nucleic acid activity and thereby is the reverse complementary sequence,
      under conditions which allow primer initiated nucleic acid amplification and an activity of a catalytic nucleic acid activity, whereby the oligonucleotide (or the primer) and the zymogen are oriented to one another such that in the presence of the nucleic acid to be examined that a nucleic acid is generated, which comprises a sequence or a reverse complementary sequence of the nucleic acid to be examined, as well as, the catalytic nucleic acid activity, and
c) the detection of the catalytic nucleic acid activity through which a presence of a methylation pattern in the nucleic acid to be examined is determined.

The method according to the invention comprises preferably as the fourth step the quantitative determination of the catalytic nucleic acid activity. For this, the use of a substrate is preferred that is converted through the catalytic nucleic acid activity. The quantification of the catalytic nucleic acid activity is then carried out through the quantification of the converted substrate. There are multiple methods known to the person skilled in the art to quantify catalytic nucleic acid activity or converted substrates. These are carried out, for example, but not limited to, through the use of one or more standards. Another possibility, and likewise preferred, is the quantification through real-time methods (an overview over the appropriate quantification methods can be obtained through PCT/EP2005/003793; Real-time PCR: An Essential Guide, Horizon Bioscience, Kirstin Edwards, Julie Logan and Nick Saunders, May 2004 ISBN: 0-9545232-7X; Real-time PCR, M. Tevfik Dorak, Taylor & Francis, April 2006), ISBN: 041537734X; Mackay IM, Arden KE, Nitsche A. Real-time PCR in virology. Nucleic Acids Res. 2002 Mar 15; 30(6): 1292-305; Bernard PS, Wittwer CT. Real-time PCR technology for cancer diagnostics. Clin Chem. 2002 Aug; 48(8): 1178-85). Moreover, the quantification of multiple catalytic nucleic acid activity can be performed relatively to one another. For example, the catalytic nucleic acid activity of sample A is equivalent to x-times the catalytic nucleic acid activity of sample B.

In a preferred embodiment of the method according to the invention, the nucleic acid to be examined is DNA, genomic DNA, RNA, ribosomal RNA, transfer-RNA, or mRNA. Especially preferred, the nucleic acid to be examined is genomic DNA, ribosomal RNA or transfer-RNA. In a preferred embodiment, the oligonucleotide (or the primer) that is brought into contact with the converted nucleic acid to be examined comprises DNA, RNA, PNA or a derivative of one of these three nucleic acids. In a preferred embodiment, the nucleic acid that is brought into contact with the converted nucleic acid to be examined and that comprises the zymogen, comprises DNA, RNA, PNA or a derivative of one of these three nucleic acids. In a preferred embodiment, the amplification of the converted nucleic acid comprises the use of nucleotides, ribonucleotides or derivatives thereof.

In a preferred embodiment, the detection of a methylation pattern is performed over amplification methods known to the person skilled in the art. These are based on a) RNA - RNA amplification, b) RNA - DNA reverse transcription, c) RNA - DNA amplification, d) DNA - RNA amplification, e) DNA - RNA transcription, as well as f) DNA - DNA amplification.

In a preferred embodiment, the nucleic acid amplification comprises at least one of the following: nucleotide, ribonucleotide, PNA-monomer, or an equivalent derivative.

In a preferred embodiment, one or more oligonucleotides, which serve as a primer for initiating nucleic acid amplification, comprise(s) at least one of the following: nucleotide, ribonucleotide, PNA-monomer, or an appropriate derivative. In a preferred embodiment, one or more oligonucleotides for nucleic acid amplification, comprise(s) a region, which serves as a methylation-specific primer. The exact technical data for methylation-specific primers or the feasibility of an appropriate amplification method are known to the person skilled in the art (compare: Herman et al.: Methylation-specific PCR: a novel PCR assay for methylation status of CpG islands. Proc Natl Acad Sci U S A 1996 Sep 3; 93(18): 9821-6; US 5,786,146; US 6,017,704; US 6,200,756).

In a preferred embodiment, nucleic acid amplification is performed in the presence of blocking molecules. The exact technical data are known to the person skilled in the art for methylation-specific blocking molecules or the feasibility of an appropriate amplification method (compare: WO 02/072880).

According to the invention, amplification methods are preferred from the group that comprises: PCR, SDA (single strand displacement amplification) like, for example, isothermal amplification, TMA (transcription mediated amplification) like, for example, NASBA, LCR, methods for methylation specific amplification, MSP (methylation specific PCR), nested MSP, HeavyMethyl^{™}, Methylation-sensitive Primerextension, or Ms-SNuPE (Methylation-sensitive Single Nucleotide Primer Extension). These methods are known to the person skilled-in the art. An overview can be obtained, for example, through PCT/US2006/014667. But also related methods, as known to the person skilled in the art, for nucleic acid amplification are useful according to the invention's methods.

In a preferred embodiment, the amplification is carried out as a real time variation. The combination with methylation-specific primers (MSP, nested MSP) and/or methylation-specific blocking molecules (HeavyMethyl^{™}) is preferred. An especially preferred embodiment comprises amplification using real time PCR using methylation-specific primers. Yet another especially preferred embodiment comprises amplification using real time PCR using methylation-specific blocking molecules.

The method according to the invention can be carried out fundamentally in six different variations, which can be divided into two groups: In the first group, the oligonucleotide that serves as a primer and the zymogen are arranged on different molecules. In the second group, the oligonucleotide that serves as a primer and the zymogen are arranged on the same molecule.

In a preferred embodiment, the bisulfite-converted nucleic acid to be examined interacts with a linear, ligatable, single-stranded nucleic acid that comprises the zymogen. This linear, ligatable, single-stranded nucleic acid comprises at its respective ends sequences, which can hybridize specifically with the converted nucleic acid to be examined. Such a probe is known to the person skilled-in the art as a "padlock-probe". Thereby, either one of the ends or both ends is (are) specific for the methylation pattern to be detected. If hybridization occurs, as long as no mismatch occurs on the methylation position being examined, a ligation is carried out. Thereby a so-called "Minicircle" is generated that comprises the zymogen.

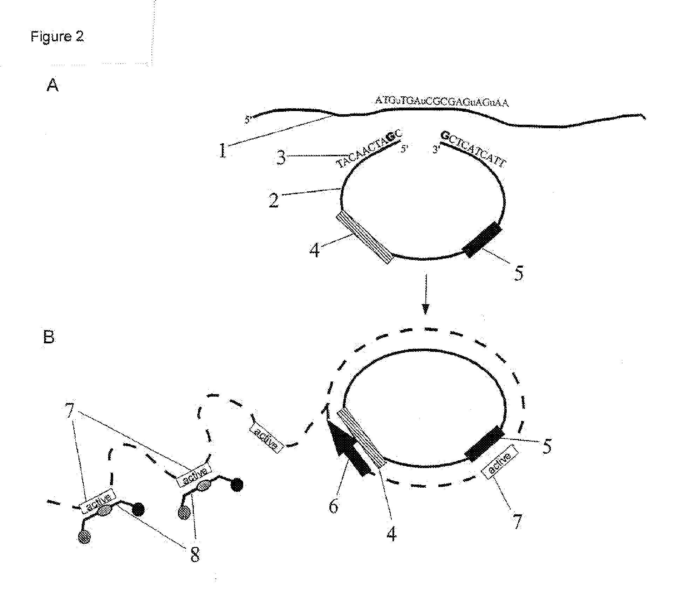

With the help of oligonucleotides, which are appropriate for the initiation of amplification, a "rolling circle"-amplification (RCA) is carried out. Thereby, said oligonucleotide binds either completely or partly with the ligated end-region or some other position of the Minicircles. The amplicon of the RCA is composed of a concatamer of the reverse complementary sequence of the Minicircles, which also serves as the template. It therefore also comprises the catalytic nucleic acid activity in multiple copies. This catalytic nucleic acid activity produces through the conversion of a substrate a detectable signal through which the methylation pattern of a nucleic acid is determined. The signal increases linearly as a function of time. The detection allows one to draw conclusions on the present methylation pattern of the nucleic acid to be examined. For a detailed description of this embodiment please refer to Figure 2.

In another preferred embodiment, a fragmentation of the nucleic acid to be examined is carried out. This is carried out either before or after the bisulfite conversion with help from physical, chemical, or biological methods: for example, but not limited to, through ultrasound treatment, shearing or digestion with restriction enzymes. In a preferred embodiment, one or more methylation-unspecific restriction enzymes are used. In another preferred embodiment, one or more methylation-unspecific restriction enzymes are used with one or more methylation-specific restriction enzymes. In a further preferred embodiment, methylation-specific restriction enzymes are used. In a preferred embodiment, at least two different methylation-specific restriction enzymes are used. These allows in the following for a direction-specific ligation.

In a preferred embodiment, the ligation of the bisulfite-converted, fragmented nucleic acid is carried out with at least one nucleic acid molecule, which comprises a zymogen. Thereby, either a circular or a linear molecule is generated that displays a constant ratio between zymogen and the fragmented converted nucleic acid. The amplification is then carried out using at least one primer, which specifically hybridizes on the ligation product. In a preferred embodiment, the primer is specific for a methylation pattern. In another preferred embodiment, the specificity for a methylation pattern is obtained through the appropriate choice of one or more methylation-specific restriction enzymes. In each case, the amplification follows through with an amplification of a zymogen-comprising segment, through which the catalytic nucleic acid activity is generated. By using a substrate, a signal is generated that allows one to draw conclusions on the existing methylation pattern of the nucleic acid to be examined.

In another preferred embodiment, the ligation of adaptors is carried out on the bisulfite-converted fragmented nucleic acid. Alternatively, the adaptors can also be produced using a terminal transferase. This enzyme adds nucleotides at the ends of the bisulfite-converted fragmented nucleic acid. The amplification is carried out with at least one primer, which completely or in part hybridizes with one or both linkers or to the converted nucleic acid. At least one of the primers for amplification comprises additionally the zymogen, so that through amplification the catalytic nucleic acid activity is generated. Through the conversion of a substrate using the catalytic nucleic acid activity, a signal is generated. According to this embodiment, the specificity for a methylation pattern is reached through the appropriate choice of one or more methylation-specific restriction enzymes.

In an especially preferred and advantageous embodiment, the converted nucleic acid to be examined is brought into contact with at least two oligonucleotides serving as primers. Thereby, at least one of the at least two oligonucleotides comprises at the same time a zymogen. Preferably, this zymogen is located 5' to the sequence functioning as a primer. Preferably, both oligonucleotides each comprise a zymogen. In an especially preferred embodiment, the detection of the catalytic nucleic acid activity generated through the amplification is performed by way of modification of a substrate, which itself is not a component of the amplificate. The confirmation of the detectable methylation pattern is carried out then through the detection of the modified substrate. Preferably, a quantification of the substrate is carried out, through which the presence of the methylation pattern is quantified. In another especially preferred embodiment, a substrate is modified through the amplification generated catalytic nucleic acid activity where the substrate itself is a component of the amplificate. Preferentially, this substrate is introduced into the amplificate through a primer. Furthermore, it is preferred that the catalytic nucleic acid activity is flanked by at least one methylation-specific area. This region or regions are located either 5' and/or 3' from of the catalytic nucleic acid activity of the amplificate. This region or regions are specific for a methylation pattern whose presence should not be detected. It follows after a depletion of background amplificates, i.e. the amplification is nearly fully disabled (each amplification primer comprises a zymogen) or is carried out only linearly (only one primer comprises a zymogen). The actual detection of the presence of the sought-after methylation pattern is carried out through the appropriate labeling of the amplificates, which are not depleted. This is carried out, for example, through fluorescence labeling of the oligonucleotides used as a primer. This embodiment is used preferentially to detect a methylation pattern in a mixture of different nucleic acids.

In an especially preferred and advantageous embodiment, the converted nucleic acid to be examined is brought into contact with at least two oligonucleotides serving as primers, which comprises no zymogen or a catalytic nucleic acid activity. Preferably, it concerns converted nucleic acid to be examined in a mixture of different converted nucleic acids. Preferably, at least one of the oligonucleotides comprises in its 3' terminal region a substrate of the catalytic nucleic acid activity. The amplification is carried out in the presence of a catalytic nucleic acid activity, whose 3' end is preferably not appropriate for the initiation of amplification. The catalytic nucleic acid activity recognizes a sequence of an oligonucleotide, which comprises a primer. In addition, it recognizes in a methylation-specific manner an amplified section of a converted nucleic acid. The successful recognition of both regions in an amplificate or in a converted nucleic acid drives it to its restriction. The restriction is carried out preferably between the positions n and n+1, n-1 and n, n-2 and n-3, n-3 and n-4, n-5 and n-6 as well as n-6 and n-7 whereas n represents the last 3' terminal base of the primers used for amplification or the corresponding position of the converted nucleic acid. Through the restriction of the amplificates, only a linear amplification of the corresponding converted nucleic acid is carried out. If the restriction of the converted nucleic acid occurs, the amplification is discontinued largely. Also, in this embodiment, the detection of a methylation pattern is carried out through the depletion of background amplificate. The actual detection of the presence of the sought-after methylation pattern carried out through the appropriate labeling of the amplificate, which is not depleted. This is carried out, for example, through the fluorescence labeling of the oligonucleotide used as a primer.

In an especially preferred embodiment, one of the mentioned six main embodiments is made with methylation-specific primers and/or with methylation-specific blockers.

According to a preferred embodiment, the catalytic nucleic acid activity is a Ribozyme or a DNAzyme. According to the invention, it is possible that the Ribozyme or the DNAzyme comprise nucleotide derivatives.

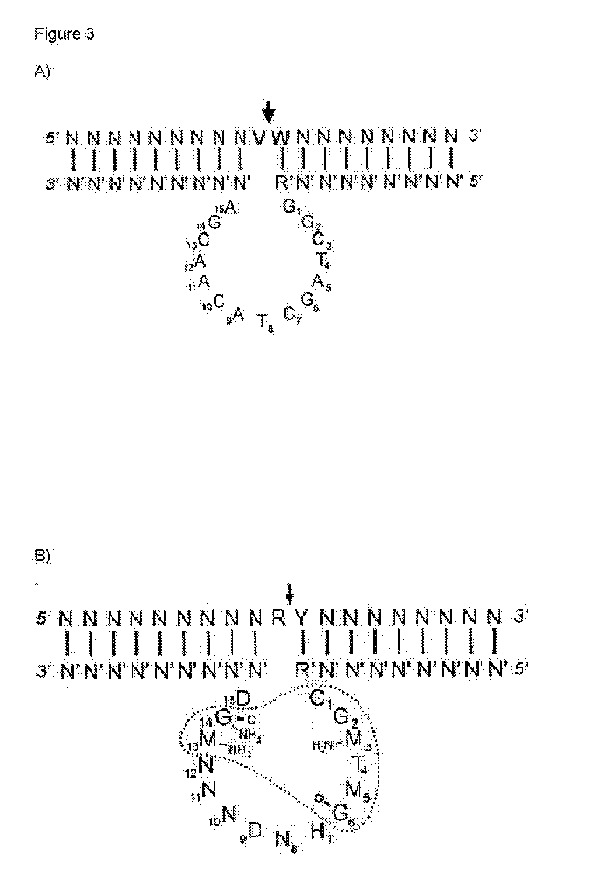

In a preferred embodiment of the invention, the catalytic nucleic acid activity is a 10-23 DNAzyme, as described, for example, in US 6,140,55. The requirements for a sequence of the catalytic domain of 10-23 DNAzyme permit different sequences for the construction of the sequence of the 10-23 DNAzyme. The possible sequences are likewise preferred and described in Figure 3. Additionally, they are also known to the person skilled-in the art (Zaborowska et al., 2002, Sequence Requirements in the Catalytic Core of the 10-23 DNA Enzyme, 277: 40617-40622).

In a preferred embodiment, the detection of a catalytic nucleic acid activity or the depletion of the background amplification through the catalytic nucleic acid activity comprises the detectable modification of at least one substrate. Preferably, the detectable modification is chosen from a group comprising: the formation and cleavage of a phosphodiester bond; nucleic acid ligation and cleavage; porphyrin metallation; formation of a carbon-carbon, ester, and amide bond. Preferentially, the detectable modification comprises the cleavage of a fluorescence labeled nucleic acid molecule, as are known to the person skilled in the art.

In a preferred embodiment, the substrate is a DNA-RNA chimera. This is especially preferred, when the catalytic nucleic acid activity is a DNAzyme or a Ribozyme. In another preferred embodiment, the substrate is DNA. This is especially preferred when the catalytic nucleic acid activity is a Ribozyme. In another preferred embodiment, the substrate is RNA. This is especially preferred, when the catalytic nucleic acid activity is a DNAzyme.

In a preferred embodiment, the substrate is a part of the amplificate. The catalytic nucleic acid activity converts, in particular cleaves, the substrate or amplificate. Thereby, a decrease in the undesirable amplificates is attained, which cannot be further amplified. The desirable amplificates can then be better amplified and detected, according to the embodiment described herein or conventional methods. Preferably, the building-in of the substrates into the amplificate is carried out through a primer of the amplification.

In a preferred embodiment, the substrate is modified at its 3' end such that it is no longer suitable for the initiation of the amplification. The person skilled in the art would know the appropriate modifications. For example, but not limited to, the modification is a 3' terminal dideoxynucleotide. Preferably, an amplification enzyme is used that possesses no 3'-5' exonuclease activity.

The substrate is converted in a preferred embodiment by the catalytic nucleic acid activity, in particular cleaved. A detectable signal is generated at the cleavage such that the quantification of the catalytic nucleic acid activity and with that the detection of the presence of the methylation pattern is permitted.

In a preferred embodiment, the substrate is labeled. The person skilled in the art would know the appropriate labels. Preferably, the labeling is carried out through radioactivity, Biotin, antibodies, ligands, mass labeling, or fluorescent dye. For example, but not limited to, in real time based amplification methods, the detection of converted or not converted substrates can be performed where the substrate is immobilized and the catalytic nucleic acid activity carries out the separation of the label. Labels still attached would mean then the substrate is not converted while separated labels would mean converted substrates.

In a preferred embodiment, the substrate is labeled with a pair of fluorophor and quencher. Preferably, at one end a fluorophor and at the other end a quencher is arranged. Suitable fluorophors and quenchers are known to the person skilled-in the art. For example, but not limited to, in Table 1, suitable fluorophors and quenchers are given. Preferably, the fluorophors FAM, HEX, ROX und Tamra, and the Quenchers BHQ1 and Dabcyl are used. Through this kind of labeling a detectable signal is first produced during the conversion of the substrate by the catalytic nucleic acid activity. This labeling technique is particularly preferred in real-time based embodiments of the method according to the invention.

**Table 1**

| **Dye** | **Em** | **Ext. Coeff.** | **Quencher** | | | | | |
|---|---|---|---|---|---|---|---|---|
| Cascade Blue | 410 | | Dabcyl | QSY-35 | | | | |
| 7-Methoxycoumarin | 410 | | Dabcyl | QSY-35 | | | | |
| Alexa Fluor 350 | 442 | 19000 | Dabcyl | QSY-35 | | | | |
| AMCA | 442 | 19000 | Dabcyl | QSY-35 | | | | |
| Coumarin(AMCA-X) | 442 | | Dabcyl | QSY-35 | | | | |
| Pacific Blue | 451 | 37000 | Dabcyl | QSY-35 | | | | |
| Marina Blue | 459 | 19000 | Dabcyl | QSY-35 | | | | |
| Dialkylamino-coumarin | 468 | | Dabcyl | QSY-35 | | | | |
| BODIPY 493/503 | 509 | 79000 | Dabcyl | TAMRA | BHQ-1 | QSY-7 | QSY-9 | |
| BODIPY-FL-X | 510 | | Dabcyl | TAMRA | BHQ-1 | QSY-7 | QSY-9 | |
| BODIPY FL | 510 | 70000 | Dabcyl | TAMRA | BHQ-1 | QSY-7 | QSY-9 | |
| DTAF (5-DTAF) | 516 | | Dabcyl | TAMRA | BHQ-1 | QSY-7 | QSY-9 | |
| 6-FAM (Fluorescein) | 516 | | Dabcyl | TAMRA | BHQ-1 | QSY-7 | QSY-9 | |
| Oregon Green 488 | 516 (521) | 76000 | Dabcyl | TAMRA | BHQ-1 | QSY-7 | QSY-9 | |
| Dansyl-X | 518 | | Dabcyl | TAMRA | BHQ-1 | QSY-7 | QSY-9 | |
| Alexa Fluor 488 | 519 | 71000 | Dabcyl | TAMRA | BHQ-1 | QSY-7 | QSY-9 | |
| Oregon Green 500 | 519 | 84000 | Dabcyl | TAMRA | BHQ-1 | QSY-7 | QSY-9 | |
| FAM | 520 | 83000 | Dabcyl | TAMRA | BHQ-1 | QSY-7 | QSY-9 | |
| FITC | 520 | 73000 | Dabcyl | TAMRA | BHQ-1 | QSY-7 | QSY-9 | |
| Fluorescein | 520 | 83000 | Dabcyl | TAMRA | BHQ-1 | QSY-7 | QSY-9 | |
| Rhodol Green | 523 | | Dabcyl | TAMRA | BHQ-1 | QSY-7 | QSY-9 | |
| Oregon Green 514 | 526 | 85000 | Dabcyl | TAMRA | BHQ-1 | QSY-7 | QSY-9 | |
| Rhodamine Green | 528 | 74000 | Dabcyl | TAMRA | BHQ-1 | QSY-7 | QSY-9 | |
| Rhodamine Green-X | 528 | 74000 | Dabcyl | TAMRA | BHQ-1 | QSY-7 | QSY-9 | |
| NBD | 535 | | Dabcyl | TAMRA | BHQ-1 | QSY-7 | QSY-9 | |
| TET | 536 | 73000 | Dabcyl | TAMRA | BHQ-1 | QSY-7 | QSY-9 | |
| Alexa Fluor 430 | 541 | 16000 | Dabcyl | TAMRA | BHQ-1 | QSY-7 | QSY-9 | |
| BODIPY- FLBR2 | 545 | | TAMRA | BHQ-1 | QSY-7 | QSY-9 | | |
| Bodipy- R6G | 547 | 70000 | TAMRA | BHQ-1 | QSY-7 | QSY-9 | | |
| JOE | 548 | 73000 | TAMRA | BHQ-1 | QSY-7 | QSY-9 | | |
| 6-JOE | 548 | 73000 | TAMRA | BHQ-1 | QSY-7 | QSY-9 | | |
| BODIPY 530/550 | 551 | | TAMRA | BHQ-1 | QSY-7 | QSY-9 | BHQ-2 | |
| HEX | 556 | 73000 | TAMRA | BHQ-1 | QSY-7 | QSY-9 | BHQ-2 | |
| Carboxyrhodamine 6G | 557 | | BHQ-1 | QSY-7 | QSY-9 | BHQ-2 | | |
| Alexa Fluor 555 | 565 | 150000 | BHQ-1 | QSY-7 | QSY-9 | BHQ-2 | | |
| BODIPY 558/568 | 568 | | BHQ-1 | QSY-7 | QSY-9 | BHQ-2 | | |
| BODIPY 564/570 | 569 | 142000 | BHQ-1 | QSY-7 | QSY-9 | BHQ-2 | | |
| BODIPY TMR | 570 | 56000 | BHQ-1 | QSY-7 | QSY-9 | BHQ-2 | | |
| BODIPY-TMR-X | 570 | | BHQ-1 | QSY-7 | QSY-9 | BHQ-2 | | |
| PyMPO | 570 | | BHQ-1 | QSY-7 | QSY-9 | BHQ-2 | | |
| Cy3 | 570 | 150000 | BHQ-1 | QSY-7 | QSY-9 | BHQ-2 | | |
| TAMRA | 576 | 90000 | BHQ-1 | QSY-7 | QSY-9 | BHQ-2 | | |
| Rhodamine Red | 580 | 129000 | BHQ-1 | QSY-7 | QSY-9 | BHQ-2 | | |
| BODIPY 576/589 | 589 | | BHQ-1 | QSY-7 | QSY-9 | BHQ-2 | DDQII | QSY-21 |
| BODIPY 581/591 | 591 | 136000 | BHQ-1 | QSY-7 | QSY-9 | BHQ-2 | DDQII | QSY-21 |
| QSY-7 | 591 | | BHQ-1 | QSY-7 | QSY-9 | BHQ-2 | DDQII | QSY-21 |
| Cy3.5 | 596 (604) | 150000 | BHQ-1 | QSY-7 | QSY-9 | BHQ-2 | DDQII | QSY-21 |
| ROX | 602 (605) | 82000 | BHQ-1 | QSY-7 | QSY-9 | BHQ-2 | DDQII | QSY-21 |
| Texas Red-X | 603 | | QSY-7 | QSY-9 | BHQ-2 | DDQII | QSY-21 | |
| Texas Red | 603 | 116000 | QSY-7 | QSY-9 | BHQ-2 | DDQII | QSY-21 | |
| BODIPY TR | 616 | 68000 | BHQ-2 | DDQII | QSY-21 | BHQ-3 | | |
| BODIPY 630/650 | 640 | 101000 | BHQ-2 | DDQII | QSY-21 | BHQ-3 | | |
| Alexa Fluor 647 | 665 | 239000 | BHQ-2 | DDQI | QSY-21 | BHQ-3 | | |
| BODIPY 650/665 | 665 | 101000 | BHQ-2 | DDQII | QSY-21 | BHQ-3 | | |
| Cy5 | 667 (670) | 250000 | BHQ-2 | DDQII | QSY-21 | BHQ-3 | | |
| Alexa Fluor 660 | 690 | 132000 | DDQII | QSY-21 | BHQ-3 | | | |
| Cy5.5 | 694 | 250000 | DDQII | QSY-21 | BHQ-3 | | | |

Table 1 shows an overview of the preferred embodiments where a substrate is labeled with a fluorophor or a quencher. The table shows preferred combinations of fluorophors and quenchers. Dabcyl functions as a quencher in a region from 380-530 nm; QSY-35 functions as a quencher in the region of 410-500 nm; BHQ1 (Black Hole Quencher 1) functions as a quencher in the region of 480-580 nm; TAMRA functions as a quencher in region of 500 nm-550 nm; QSY-7 functions as a quencher in the region of 500-600 nm; QSY-9 functions as a quencher in the region of 500-600 nm; BHQ-2 (Black Hole Quencher 2) functions as a quencher in the region of 550-650 nm; DDQII (Deep Dark Quencher II) functions as a quencher in the region of 590 nm-700 nm; QSY-21 functions as a quencher in the region of 590 nm-720 nm; and BHQ-3 (Black Hole Quencher 3) functions as a quencher in the region of 620 nm-730 nm. It is known to the person skilled in the art that further fluorophors or quenchers are also applicable are also hereby preferred.

In a preferred embodiment, the methylation-specific amplification of the nucleic acid to be examined is carried out after the bisulfite conversion. Only after that, the methylation-specific amplification, comprising a zymogen and/or catalytic nucleic acid activity, is carried out. Preferably, the methylation-unspecific amplification is region-unspecific. This is also known as the "whole genome" amplification. The person skilled in the art would know the appropriate methods. Especially preferred are methods, which are especially well suited for the whole genome amplification of bisulfite-converted DNA. Preferentially, the whole genome amplification is carried out as described in PCT/US2006/014667. In another preferred embodiment, the methylation-unspecific amplification is carried out in a region-specific manner. The amplification is carried out here through the choice of the appropriate primer. Depending on the questions, it is advantageous to amplify only one region or multiple regions of the converted nucleic acid.

In a further embodiment, an enrichment of the nucleic acid to be examined is carried out. This can be done either before, as well as, after the bisulfite conversion. The person skilled in the art would know the appropriate methods. Preferably, the enrichment is carried out on methylated and/or unmethylated nucleic acid before the bisulfite treatment, essentially as described in PCT/US2006/005379. Preferably, the enrichment of the bisulfite-treated nucleic acid is carried out through either a gene-specific or gene-unspecific amplification, particularly through gene-specific PCR or through a so-called "Whole Genome Amplification" (WGA; for example analogous to PCT/US2006/014667).

In a preferred embodiment, the detection of multiple methylation patterns is carried out. This embodiment comprises the conversion of a nucleic acid to be examined, so that 5-methylcytosine remains unchanged while unmethylated cytosine is converted into uracil or into another base, which can be distinguished from cytosine in its base-pairing behavior. It comprises, in addition, multiple zymogens. Hereby, at least one zymogen is allotted to one methylation pattern. This at least one zymogen is characterized through its encoded catalytic nucleic acid activity that permits it to be clearly distinguished from other catalytic nucleic acid activities, which are allotted to other methylation patterns. Furthermore, this embodiment comprises the detection of a variety of catalytic nucleic acid activity, which has been generated through an amplification of the converted nucleic acid in the presence of the zymogens.

An especially preferred embodiment comprises multiple oligonucleotides. Hereby, there is at least one oligonucleotide specific for one methylation pattern. Said oligonucleotides are suitable for initiating nucleic acid amplification. During the amplification, an oligonucleotide is oriented to its corresponding zymogen so that an amplificate is generated that comprises a sequence or a reverse complementary sequence of the converted nucleic acid to be examined, as also the catalytic nucleic acid activity encoded by the zymogen.

An especially preferred embodiment comprises the quantification of multiple methylation patterns. This is carried out through the quantification of the catalytic nucleic acid activities generated by amplification.

In a preferred embodiment, the detection of multiple methylation patterns is performed in the presence of different nucleic acids. This embodiment comprises the conversion of the different nucleic acid so that 5-methylcytosine remains unchanged while the unmethylated cytosine is converted into uracil or into another base, which can be distinguished from cytosine in its base-pairing behavior. In addition, it comprises of at least one oligonucleotide suitable for the initiation of nucleic acid amplification. Furthermore, it comprises at least one catalytic nucleic acid activity, which is able to modify a fraction of the amplificates such that they are no longer available for further amplifications. These catalytic nucleic acid activities are specific for methylation patterns no to be detected. Preferably, it concerns methylation patterns that comprise the same cytosine position, the same cytosine positions, or only a part thereof of different methylation. In this way, there is a repression of amplification of amplificates, which are indicative of undesirable methylation patterns. The presence of the desired methylation pattern results from the detection of the amplificates whose amplification is not repressed. This detection is performed according to standard methods known to the person skilled in the art. The present methylation patterns are also quantifiable. The person skilled in the art would know the appropriate methods for this.

In a preferred embodiment, the catalytic nucleic acid activities are encoded through zymogens that are located on the oligonucleotides, which also comprise a region serving as a primer. Therefore, a catalytic nucleic acid activity is part of the amplificate. A catalytic nucleic acid activity is able to bind methylation specifically and modify the amplificate.

Preferably, this is carried out in *cis*: that is, a catalytic nucleic activity modifies the amplificate that it itself comprises.

In another preferred embodiment, the catalytic nucleic acid activities do not result from the amplification of the converted nucleic acid. Preferably, they are added to the amplification. The catalytic nucleic acid activity, in this embodiment, is not only able to modify the amplificate, but also a portion of the converted nucleic acid to be examined. As a result, only a fraction of the converted nucleic acid to be examined or only a fraction of the amplificate is available for further amplification.

In a preferred embodiment, one or more detectable or quantifiable methylation patterns are indicative of disease.

The method according to the invention differs from other methods known to date in that it permits a higher sensitivity and specificity in the detection of one or more methylation patterns of a nucleic acid or a mixture of different nucleic acids. Hereby, a methylation pattern comprises one or more positions in a nucleic acid, which are methylated or not methylated, respectively. A distinguishable feature of the embodiments of the method according to the invention is that the analysis of nucleic acids (nucleic acids to be examined or converted nucleic acids to be examined) smaller than 200 nucleotides (nt) is permitted. Preferentially, a distinguishable feature of the embodiments is that the analysis of nucleic acids (nucleic acids to be examined or converted nucleic acids to be examined) smaller than 100 nt is permitted. Surprisingly, the method according to the invention comprises also embodiments where the distinguishable feature is that the analysis of nucleic acids (nucleic acids to be examined or converted nucleic acids to be examined) smaller than 50 nt is permitted.

A part of the invention is also an oligonucleotide, which possesses a higher sensitivity or specificity in detecting one or more methylation patterns in a nucleic acid or in a mixture of different nucleic acids than previously known oligonucleotides. Hereby, a methylation pattern of one or more positions of a nucleic acid comprises the presence of said positions that are methylated or not methylated, respectively. According to the invention, the oligonucleotide comprises a region, which comprises the bases A, C, T, G, or a derivative thereof in positions, which are suitable for specifically recognizing individual methylation patterns. These positions have methylation specificity as well as sequence specificity. In addition, the region comprises only the bases A, C, T, or their derivatives, or bases A, G, T, or their derivatives in all other positions. These possess sequence specificity. Preferably, this region is located in the 3' end of the oligonucleotide according to the invention. The oligonucleotide according to the invention further comprises a catalytic nucleic acid activity or a sequence, which encodes a catalytic nucleic acid activity.

A preferred oligonucleotide is characterized in that it is capable of initiating an extension or amplification. Preferably, such an oligonucleotide is used in an embodiment of the invention, in which the catalytic nucleic acid activity is produced during the amplification.

Another preferred oligonucleotide is that it cannot be extended, thereby cannot be amplified. Preferably, such an oligonucleotide is used in an embodiment of the invention, in which the amplification is carried out where the amplification is indicative of a methylation pattern that is not to be detected.

A preferred oligonucleotide comprises further at least one region that is specific for a substrate or for a reverse complementary sequence of a substrate. This region or these regions are located preferably 5' and/or 3' of the catalytic nucleic acid activity or 5' and/or 3' of the sequence that encodes a catalytic nucleic acid activity.

The invention comprises further a kit comprising a reagent or enzyme for the conversion of a nucleic acid to be examined so that that 5-methylcytosine remains unchanged while the unmethylated cytosine is converted into uracil or into another base, which can be distinguished from cytosine in its base-pairing behavior. The kit according to the invention comprises further a catalytic nucleic acid activity or a sequence, which encodes a nucleic acid activity. A preferred embodiment of the kit comprises, in addition, a repository in which all the components of the kit can be placed.

An especially preferred embodiment of the kit comprises a region of an oligonucleotide, which is suitable for initiating the nucleic acid amplification. Preferably, said region is suited to function as a methylation-specific primer.

An especially preferred embodiment of the kit comprises a methylation-specific blocker molecule.

An especially preferred embodiment of the kit comprises a substrate, which is modified through the catalytic nucleic acid activity in a detectable manner. Preferably, the substrate is a nucleic acid. Preferably, the substrate is labeled. Preferably, the substrate is labeled with a fluorescent-dye, particularly with a fluorescent-dye and a quencher.

An especially preferred embodiment of the kit comprises one or more oligonucleotides according to the invention. An especially preferred embodiment of the kit comprises one or more methylation-specific primers. An especially preferred embodiment of the kit comprises one or more oligonucleotides, which comprise a region that functions as a methylation-specific primer.

An especially preferred kit is a kit for nucleic acid amplification. Preferably, it concerns a method, as it is known to the person skilled-in the art. Especially preferred, it concerns a method selected from a group comprising: PCR, SDA (single strand displacement amplification) like, for example, isothermal amplification, TMA (transcription mediated amplification) like, for example, NASBA, LCR, methods for methylation specific amplification, MSP, (methylation specific PCR), nested MSP, HeavyMethyl^{™}, Methylation-sensitive Primerextension, or Ms-SNuPE (Methylation-sensitive Single Nucleotide Primer Extension).

Preferably, the invention further comprises the use of the method according to the invention, of the oligonucleotide and of the kit according to the invention for diagnosis and/or prognosis of adverse events in patients or individuals. An adverse event is hereby an event that belongs to one or more of the following categories: Undesired drug side effects; cancer; CNS-malfunction, impairment, disease; symptoms of aggression or behavioral disorders; clinical, psychological and social consequences from brain impairment; psychotic disorders and personality disorders; Dementia and/or associated syndromes; cardiovascular disease, malfunction and impairment; malfunction, impairment or disease of the gastrointestinal tract; malfunction, impairment or disease of the lung system; damage, inflammation, infection, immune and/or convalescence; malfunction, impairment, or disease of the body as an abnormality in development processes; malfunction, impairment, or disease of the skin, muscle, connective tissue or skeletal tissue; endocrine or metabolic malfunction, impairment or disease; headaches, or sexual malfunction.

The term "diagnosis" being used here comprises the diagnosis of an adverse event, a predisposition for an adverse event, and/or a progression of an adverse event.

Preferably, the invention further comprises the use of the method according to the invention, of the oligonucleotide and of the kit according to the invention to distinguish between cell- or tissue-types or to investigate cell-differentiation.

The method according to the invention is a method to detect the presence of one or more methylation patterns, comprising
a) the conversion of nucleic acid, so that 5-methylcytosine remains unchanged, while unmethylated cytosine is converted into uracil or another base, which can be distinguished from cytosine in its base-pairing behavior, and
b) a catalytic nucleic acid activity.

A preferred embodiment of the method according to the invention is a method to detect the presence of a methylation pattern in a sample nucleic acid, comprising
a) the conversion of a sample nucleic acid to be examined, so that 5-methylcytosine remains unchanged, while unmethylated cytosine is changed to uracil or another base, which can be distinguished from cytosine in its base-paring behavior,
b) the subjecting of the nucleic acid to be examined to
   i) an oligonucleotide suitable for initiating a nucleic acid amplification, and
   ii) a zymogen, encoding a nucleic acid catalytic activity and thereby having its reverse complementary sequence,
      under conditions, which permit a primer-initiated nucleic acid amplification and an activity of a catalytic nucleic acid activity, whereby the oligonucleotide and the zymogen are oriented with one another such that in the presence of the nucleic acid to be examined that an amplificate is generated, which comprises the sequence or the reverse complementary sequence of the nucleic acid to be examined as well as the catalytic nucleic acid activity, and
c) the detection of the catalytic nucleic acid activity through which the presence of a methylation pattern in the nucleic acid to be examined is determined.

A preferred embodiment of the method according to the invention is a method to detect the presence of a methylation pattern in a mixture of different nucleic acids, comprising
a) the conversion of a sample nucleic acid to be examined, so that 5-methylcytosine remains unchanged, while unmethylated cytosine is changed to uracil or another base, which can be distinguished from cytosine in its base-paring behavior;
b) the subjecting of the nucleic acid to be examined to at least one oligonucleotide suitable for initiating a nucleic acid amplification;
c) the subjecting of a catalytic nucleic acid activity to amplificates during the nucleic acid amplification, whereby a portion of the amplificate is modified through the catalytic nucleic acid activity;
d) the detection of the unmodified amplificates through which the presence of a methylation pattern in a mixture of different nucleic acids to be examined is determined.

A preferred embodiment comprises further the quantitative determination of the unmodified amplificates through which the occurrence of a methylation pattern in the sample nucleic acid is quantified.

Preferred is an embodiment in which the quantitative determination of the catalytic nucleic acid activity comprises
a) the comparison of catalytic nucleic acid activity with a standard,
b) real-time quantification, or
c) the quantification of multiple catalytic nucleic acid activities relative to each other.

Preferred is an embodiment whereby the oligonucleotide, which comprises a primer sequence, and a zymogen are arranged on different molecules or on the same molecule.

Preferred is an embodiment wherein the nucleic acid amplification is a "rolling circle" amplification.

A preferred embodiment comprises the fragmentation of the DNA being examined with one or more restriction enzymes.

A preferred embodiment comprises additionally the ligation of adaptors on to the produced fragments.

A preferred embodiment comprises additionally the ligation of zymogens to the produced fragments.

Preferred is an embodiment in which the converted nucleic acid is brought into contact with at least two oligonucleotides, each comprising a primer sequence, and additionally, at least one of the oligonucleotide comprises a zymogen.

Preferred is an embodiment in which the catalytic nucleic acid activity of an amplificate recognizes in *cis* a sequence of the amplificate and cuts the amplificate.

Preferred is an embodiment in which the nucleic acid to be examined is brought into contact with at least two oligonucleotides and said oligonucleotides each comprise a primer sequence and a zymogen.

Preferred is an embodiment where the nucleic acid to be examined is genomic DNA, DNA or RNA.

A preferred embodiment comprises the reverse transcription of the RNA to be examined.

A preferred embodiment comprises the transcription of the DNA to be examined.

Preferred is an embodiment where one or more oligonucleotides comprise at least one nucleotide, a ribonucleotide, PNA-monomer, or their derivatives.

Preferred is an embodiment where the use of nucleic acid amplification comprises at least one nucleotide, a ribonucleotide, PNA-monomer, or their derivatives.

Preferred is an embodiment where the catalytic nucleic acid activity is a Ribozyme or a DNAzyme.

Preferred is an embodiment where the catalytic nucleic acid activity comprises a detectable modification of at least one substrate.

Preferred is an embodiment where the detectable modification will be chosen from a group comprising: the formation and cleavage of a phosphodiester bond; nucleic acid ligation and cleavage; porphyrin metallation; formation of carbon-carbon-, ester-, and amide- bonds.

Preferred is an embodiment where the fluorescent-labeled nucleic acid molecule is a DNA-RNA chimera.

Preferred is an embodiment where at least one oligonucleotide comprises a region, which functions as methylation-specific primer.

A preferred embodiment comprises, in addition to all embodiments mentioned herein, methylation-specific blocker molecules.

A preferred embodiment comprises an additional methylation-specific or methylation-specific amplification of the converted DNA.

Preferably the method according to the invention is a method to detect the presence of multiple methylation patterns in the sample nucleic acid, comprising
a) the conversion of a sample nucleic acid to be examined, so that 5-methylcytosine remains unchanged, while unmethylated cytosine is changed to uracil or another base, which can be distinguished from cytosine in its base-paring behavior,
b) the subjecting of the nucleic acid to be examined to
   i) multiple oligonucleotides, wherein for each methylation pattern there is at least one corresponding oligonucleotide, which is suitable for initiating a nucleic acid amplification; and
   ii) multiple zymogens, wherein for each methylation pattern there is at least one corresponding zymogen which encodes a catalytic nucleic acid activity whereby its activity is separately measurable and which is its reverse complementary sequence,
      under conditions, which permit the primer-initiated nucleic acid amplification and an activity of the catalytic nucleic acid activity, whereby the oligonucleotide and the zymogen allotted to a methylation pattern, are oriented to one another such that in the presence of the nucleic acid to be examined, that a nucleic acid molecule is generated, which comprises the sequence or the reverse complementary sequence of the nucleic acid to be examined as well as the catalytic nucleic acid activity, and
c) the simultaneous detection of each catalytic nucleic acid activity, whereby the presence of a methylation pattern in the nucleic acid to be examined is determined.

A preferred embodiment comprises further the quantitative determination of each catalytic nucleic acid activity generated in step b) through which the incidence of the methylation pattern in the nucleic acid sample is quantified.

Preferably the method according to the invention is a method to detect the presence of multiple methylation patterns in a mixture of different nucleic acids, comprising
a) the conversion of different nucleic acids to be examined, so that 5-methylcytosine remains unchanged, while unmethylated cytosine is changed to uracil or another base that can be distinguished from cytosine in its base-paring behavior,
b) the subjecting of the nucleic acid to be examined to at least one oligonucleotide suitable for initiating a nucleic acid amplification;
c) the subjecting of a catalytic nucleic acid activity to amplificates during a nucleic acid amplification where a portion of the amplificate is modified through the catalytic nucleic acid activity,
d) the detection of the unmodified amplificate through which the presence of a methylation pattern in a mixture of different nucleic acids to be examined is determined.

A preferred embodiment comprises further the quantitative determination of the unmodified amplificates through which the incidence of a methylation pattern in the sample nucleic acid is quantified.

Preferred is an embodiment where the conversion of the nucleic acid comprises bisulfite.

Preferred is an embodiment wherein the nucleic acid amplification is carried out according to a method from the group comprising of PCR, SDA, and TMA.

Preferred is an embodiment where the presence of a methylation pattern in a sample indicates a disease.

Preferred is further an oligonucleotide comprising
a) a sequence comprising a catalytic nucleic acid activity or a sequence which encodes a catalytic nucleic acid activity, and
b) a sequence portion, which
   i) comprises the bases A, C, T, or G in positions, which are suitable for distinguishing between converted methylated and converted unmethylated positions of the nucleic acid being examined, and
   ii) only the bases A, T, or C or only the bases A, T, or G in all other positions.

An especially preferred embodiment comprises in addition one or more sequence portions, which by itself or through the reverse complimentary sequence, mediates the binding to a substrate.

Preferred is further a kit for the implementation of the method according to the invention, comprising
a) a reagent or enzyme, which enables a conversion of the nucleic acid to be examined so that 5-methylcytosine remains unchanged, while unmethylated cytosine is changed to uracil or another base, which can be distinguished from cytosine in its base-paring behavior,
b) a catalytic nucleic acid activity or a zymogen, which encodes a catalytic nucleic acid activity.

An especially preferred kit comprises
a) an oligonucleotide suitable for the initiation of a nucleic acid amplification, and/or
b) a methylation specific blocking molecule.

An especially preferred kit comprises a substrate that is modified through the catalytic nucleic acid activity in a detectable manner.

Especially preferred is a kit wherein the region of the oligonucleotides which enables the initiation of the nucleic acid,
a) comprises the bases A, C, T, or G in positions, which are suitable for distinguishing between converted methylated and converted unmethylated positions of the nucleic acid being examined, and
b) comprises only the bases A, T, or C or only the bases A, T, or G in all other positions

A preferred use of the method according to the invention or the oligonucleotide according to the invention or the kit according to the invention, is the use in making diagnoses or prognoses for adverse events for patients or individuals where the adverse events belong to at least one of the following categories: Undesired drug side effects; cancer; CNS-malfunction, impairment, disease; symptoms of aggression or behavioral disorders; clinical, psychological and social consequences from brain impairment; psychotic disorders and personality disorders; Dementia and/or associated syndromes; cardiovascular disease, malfunction and impairment; malfunction, impairment or disease of the gastrointestinal tract; malfunction, impairment or disease of the lung system; damage, inflammation, infection, immune and/or convalescence; malfunction, impairment, or disease of the body as an abnormality in development processes; malfunction, impairment, or disease of the skin, muscle, connective tissue or skeletal tissue; endocrine or metabolic malfunction, impairment or disease; headaches, or sexual malfunction.

A preferred use of the method according to the invention or the oligonucleotide according to the invention or the kit according to the invention is their application in distinguishing between cell- or tissues types or to examine cell-differentiation.

All documents cited herein are herewith cited with reference to their entirety.

### Examples

### Example 1: Quantification of methylation of Exon1 in GSTp1-genes using real-time duplex-PCR.

*Introduction:* In this example, the methylation of Exon1 in GSTp1 gene is examined with the help of a real-time duplex-PCR. The method according to the invention permits the exact quantitative analysis of methylation status from 5 CpG positions, which are localized within a only 28 nt long sequence. The methylation-specific primers, being used for these purposes, span only a 46 nt long region, which cannot be detected by conventional TaqMan or LightCycler probes. In contrast, the detection using a zymogen-primer is independent of the length of the amplificate and can be realized in a real-time PCR. The quantification of methylated-DNA is carried out simultaneously with the quantification of the aggregate DNA with the help of a reference-PCR. For that purpose, another fragment within the GSTp1 gene is amplified. The reference-PCR is carried out in a methylation-specific manner and is also realized using a zymogen-primer. The reporter-substrates of the methylation-specific PCR and reference-PCR are provided with different fluorescent dye and can be analyzed separated from one another in real-time PCR. The relative methylation of Exon1 can be calculated from of the signal of methylated-DNA and aggregate-DNA.

*Experimental Method:* DNA is extracted from tissue samples with the help of a DNA-extraction kit (DNeasy® Tissue-Kit, Quiagen). Finally, a bisulfite conversion is carried out of the DNA according to the state of the art (e.g. WO2005038051). 10 µl of the bisulfite-converted, purified DNA is used in real-time duplex-PCR, as described here, or stored at 4 °C maximum.

1 µg CpGenome^{™} Universal Methylated DNA (Chemicon International, Temecula USA) is also converted with bisulfite and serves as standard-DNA for the quantification of methylated-DNA from tumor tissue.

For the methylation-specific real-time PCR, a region of Exon1 from the GSTp1 gene (GeneBank AY324387 nt 1877-1923) is analyzed after bisulfite conversion (GSTp1 region A; SEQ ID NO: 4). The use of a forward primer 1 (SEQ ID NO: 1) and zymogen primer 1 (SEQ ID NO: 2) results in the amplification of GSTp1-amplificate 1 (SEQ ID NO: 11). The catalytic nucleic acid activity (SEQ ID NO: 5), a DNAzyme, is a part of the amplificate and is localized on the reverse complementary strand of the amplificate sequence (SEQ ID NO: 11). It cleaves the Reporter Substrate 1 at the ribonucleotides "gu" through which the fluorophor and quencher are spatially separated from another.

For the methylation-specific real-time PCR, a region downstream of Exon1 from the GSTp1 gene is analyzed after bisulfite conversion (GSTp1 region A; SEQ ID NO: 9). The use of the forward primer 2 (SEQ ID NO: 6) and the zymogen primer 2 (SEQ ID NO: 7) results in the amplification of GSTp1-amplificate 2 (SEQ ID NO: 12). The catalytic nucleic acid activity (SEQ ID NO: 10), a DNAzyme, is a part of the amplificate and is localized on the reverse complementary strand of the amplificate sequence shown (SEQ ID NO: 12). It cleaves the Reporter Substrate 1 at the ribonucleotides "gu" through which the fluorophor and quencher are spatially separated from another.

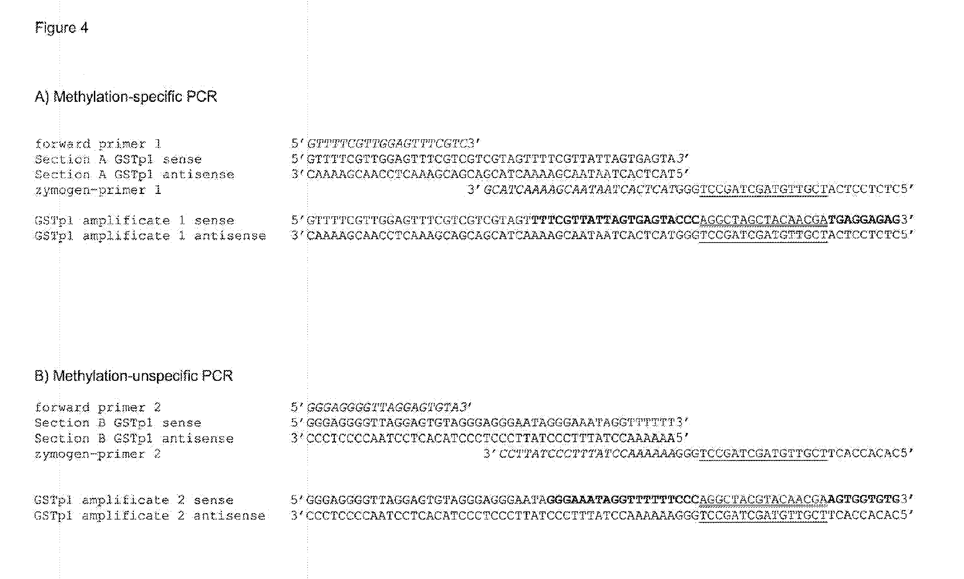

An overview of the sequences is given in Table 2. The orientation of the sequences to one another is presented in Figure 4. The primers are each specific for the bisulfite-converted sequences.

The reaction mixture (20 µl) contains the following components:
10 µl template-DNA
2 µl 10x PCR buffer (Eurogentec)
200 nmol/I dNTP (each)
3.5 mmol/l MgCl₂ (Roche Diagnostics)
0.4 µmol/l forward primer 1 (SEQ ID NO: 1)
0.1 µmol/l zymogen primer 1 (SEQ ID NO: 2)
0.2 µmol/l Reporter Substrate 1 (SEQ ID NO: 3)
0.4 µmol/l forward primer 2 (SEQ ID NO: 6)
0.1 µmol/l zymogen primer 2 (SEQ ID NO: 7)
0.2 µmol/l Reporter Substrate 2 (SEQ ID NO: 8)
1 U HotGoldStar Polymerase (Eurogentec)

The duplex real time PCR has the following temperature-time profile: Denaturing 10 min 95 °C; Amplification: 45 cycles à 10 s 95 °C, 20 s 56 °C; Cooling: 10 s at 40 °C. The Amplification is carried out in a real-time PCR apparatus (LightCycler 2.0, Roche Diagnaostics). The fluorescence signal, resulting from the converted reporter substrates, is detected at 530 nm and at 610 nm in each cycle at step 20 s 56 °C.

*Data analysis:* The fluorescence data are analyzed with help from software provided by the manufacturer and the "threshold cycle" (CT) ascertained. This is state of the art and known to the expert (i.e.. Real-time PCR, M. Tevfik Dorak, Taylor & Francis, April 2006, ISBN: 041537734X). Basically two possibilities exist for data analysis.

*Possibility 1:* An external DNA-standard dilution series is used to determine the quantity of methylated-DNA in Exon1. For that, the "threshold cycle" (CT) from channel 530 nm is used. Likewise, the determination of the aggregate DNA of the GSTp1 gene is carried out with the help of an external DNA-standard dilution series using CT from channel 610 nm. The degree of methylation in the CpG positions of Exon1 is calculated based on the ratio between the amount of methylated-DNA to the amount of aggregate-DNA (Eads CA, Lord RV, Wickramasinghe K, Long TI, Kurumboor SK, Bernstein L, Peters JH, DeMeester SR, DeMeester TR, Skinner KA, Laird PW. Epigenetic patterns in the progression of esophageal adenocarcinoma. Cancer Res. 2001 Apr 15; 61 (8):3410-8).

*Possibility 2:* The degree of methylation can also be calculated from the difference in the CT (Delta-CT) of the channel 530 nm (methylated-DNA) to the channel 610 nm (aggregate DNA). The Delta-CT value amounts to approximately zero when the DNA being examined is completely methylated. The higher the value of the Delta-CT the smaller the amount methylated-DNA is present. This type of relative calculation of the degree of methylation does not require an external DNA-standard dilution series. This methodology is state of the art and known to the expert (for example through WO 2005/098035).

**Tabelle 2: Overview of the Sequences**

| **SEQ ID NO** | **Name** | **Sequence** |
|---|---|---|
| SEQ ID NO: 1 | forward Primer 1 | *5'-GTTTTCGTTGGAGTTTCGTC-3'* |
| SEQ ID NO: 2 | zymogen-Primer 1 | 5'-CTCTCCTCATCGTTGTAGCTAGCCTGGGTACTCACTAATAACG AAAACTACG-3' |
| SEQ ID NO: 3 | Reporter Substrate 1 | 5'-FAM-CTCTCCTCAguGGGTACTCACTAATAACGAAA-BHQ1-3' |
| SEQ ID NO: 4 | GSTp1 region A (sense) | *5'-GTTTTCGTTGGAGTTTCGTCGTCGTAGTTTTCGTTATTAGTGA GTA-3'* |
| SEQ ID NO: 11 | GSTp1 Amplificate-1 (sense) | *5'-GTTTTCGTTGGAGTTTCGTCGTCGTAGTTTTCGTTATTAGTGA* **GTACCC**AGGCTAGCTACAACGA**TGAGGAGAG**-3' |
| SEQ ID NO: 5 | Sequence of methylation-specific DNAzymes | 5'-TTATTAGTGAGTACCCAGGCTAGCTACAACGATGAGGAGAG |
| SEQ ID NO: 6 | forward Primer 2 | 5'-*GGGAGGGGTTAGGAGTGTA-3'* |
| SEQ ID NO: 7 | zymogen-Primer 2 | 5'-CACACCACTTCGTTGTAGCTAGCCTGGGAAAAAACCTATTTCC *CTATTCC-3'* |
| SEQ ID NO: 8 | Reporter Substrate 2 | 5'-HEX-CACACCACTguGGG*AAAAAACCTATTTCCC*-BHQ1-3' |
| SEQ ID NO: 9 | GSTp1 region B (sense) | 5'-*GGGAGGGGTTAGGAGTGTAGGGAGGGAATAGGGAAATAGGT TTTTT-*3' |
| SEQ ID NO: 10 | Sequence of methylation-specific DNAzymes | 5'-*GGGAAATAGGTTTTTT*CCCAGGCTAGCTACAACGAAGTGGTG TG-3' |
| SEQ ID NO: 12 | GSTp1 Amplificate-2 | 5'-GGGAGGGGTTAGGAGTGTAGGGAGGGAATA**GGGAAATAGGT *TTTTT*CCC**AGGCTACGTACAACGA**AGTGGTGTG**-3' |

Italicized sequences show gene-specific sequences of the primer. Singly underlined sequences are the zymogen regions, which encode the catalytic portion of the respective DNAzymes. Doubly underlined sequences are catalytic portion of the respective DNAzymes. Upper case letters represent deoxyribonucleotides, and lower case letters represent ribonucleotides. Bold letters represent the region of the amplificate to which the respective Reporter Substrate specifically binds. The abbreviation of the modifications mean: FAM = 6-Carboxyfluoresceine, HEX = 6-Hexachlorofluoresceine, BHQ1 = BlackHoleQuencher1. (The orientation of the sequences to one another is depicted in Figure 4.)

*Detailed Description of the Methods:* The methylation-specific forward primer 1 (SEQ ID NO: 1) binds to the bisulfite-converted sense strand of the methylated sequence. The methylation-specific zymogen primer 1 (SEQ ID NO: 2) binds to the sequence which was synthesized after the first PCR cycle and contains at the 5'end besides the gene-specific sequence (italicized part of SEQ ID NO: 2), a zymogen (non-italicized part of SEQ ID NO: 2). Forward primer 1 and zymogen primer 1 each span two CpG positions and amplify only methylated-DNA. As a result, the zymogen-primer encoded DNAzyme is rendered methylation specific. The active methylation-specific DNAzyme (SEQ ID NO: 5) is generated starting from the third PCR cycle. The Reporter-Substrate 1, contained in the PCR reaction mixture, is a DNA/RNA chimera (SEQ ID NO: 3) and carries at the 5' end a fluorescent dye (FAM) and at the 3' end a quencher (BHQ1). The Reporter Substrate 1 has portions which are the complementary sequences of the DNAzyme (depicted in bold in SEQ ID NO: 11). The catalytic activity of the DNAzyme (doubly underlined in SEQ ID NO: 11) leads to a hydrolysis of the Reporter Substrate 1 through which the fluorescent dye FAM is spatially separated from the quencher BHQ1. As a result, the fluorescence increases in the 530 nm wavelength.

The forward primer 2 (SEQ ID NO: 6) binds to a bisulfite-converted sense strand downstream of Exon1 of the GSTp1 gene. The zymogen-primer 2 (SEQ ID NO: 7) binds to the sequence, which is synthesized after the first PCR cycle and possess at the 5' end a zymogen (non-italicized part of SEQ ID NO: 7), beside the gene-specific sequence (italicized part of SEQ ID NO: 7). Forward primer 2 and zymogen primer 2 each span two CpG positions and amplify methylated-DNA, as well as unmethylated-DNA. As a result, the zymogen-primer encoded DNAzyme is rendered methylation unspecific. The active methylation-specific DNAzyme (SEQ ID NO: 10) is generated starting from the third PCR cycle. The Reporter-Substrate 2, contained in the PCR reaction mixture, is a DNA/RNA chimera (SEQ ID NO: 8) and carries at the 5' end a fluorescent dye (HEX) and at the 3' end a quencher (BHQ1). The Reporter Substrate 2 has portions which are the complementary sequences of the methylation unspecific DNAzyme (depicted in bold in SEQ ID NO: 12). The catalytic activity of the methylation unspecific DNAzyme (doubly underlined in SEQ ID NO: 12) leads to a hydrolysis of the Reporter Substrate 2 through which the fluorescent dye HEX is spatially separated from the quencher BHQ1. As a result, the fluorescence increases in the 610 nm wavelength.

This real-time duplex-PCR represents a diagnostic method for detecting prostate cancer, which comprises CpG positions in Exon1 that are methylated when prostate cancer is present and unmethylated in healthy tissues (Song JZ, Stirzaker C, Harrison J, Melki JR, Clark SJ. Hypermethylation trigger of the glutathione-S-transferase gene (GSTP1) in prostate cancer cells. Oncogene. 2002 Feb 7; 21 (7): 1048-61); Nakayama M, Gonzalgo ML, Yegnasubramanian S, Lin X, De Marzo AM, Nelson WG. GSTP1 CpG island hypermethylation as a molecular biomarker for prostate cancer. J Cell Biochem. 2004 Feb 15; 91 (3): 540-52).

The previously described real-time duplex-PCR offers the following advantages over current methods for methylation analysis of GSTp1:
1. Analysis of co-methylation of exclusively five CpG positions of Exon1
2. Fast high-throughput-capable methylation analysis without an external standard, through a duplex reaction with a reference PCR.
3. An increase in sensitivity as well as in specificity because short fragments can also be detected. This is based on the fact that gene-specific regions of only about 45 nt (methylation-specific or methylation-unspecific PCR) can be detected.
4. Enabling the analysis of highly fragmented DNA, for example, DNA from formalin-fixed paraffin-embedded samples or tumor-DNA from body fluids like serum because short fragments can also be detected. This is based on the fact that gene-specific regions of only about 45 nt (methylation-specific or methylation-unspecific PCR) can be detected.

The sensitivity and also the specificity of a methylation based diagnostic tests, can be considerably improved through the use of the previously described method.

### Example 2: Quantification of methylation of Exon1 in the GSTp1 gene using real-time PCR.

The previously described PCR reaction in Example 1 is not carried out in a real time duplex reaction, but in individual real-time PCR reactions for methylated- and aggregate-DNA.

### Example 3: Quantification of methylation of Exon1 in the GSTp1 gene using PCR.

The previously described PCR reaction in Examples 1 and 2 are carried out in a conventional PCR apparatus. The analysis is carried out using endpoint determination of the fluorescent signal as elucidated in WO 2005/098035.

### Description of the Figures

Figure 1:

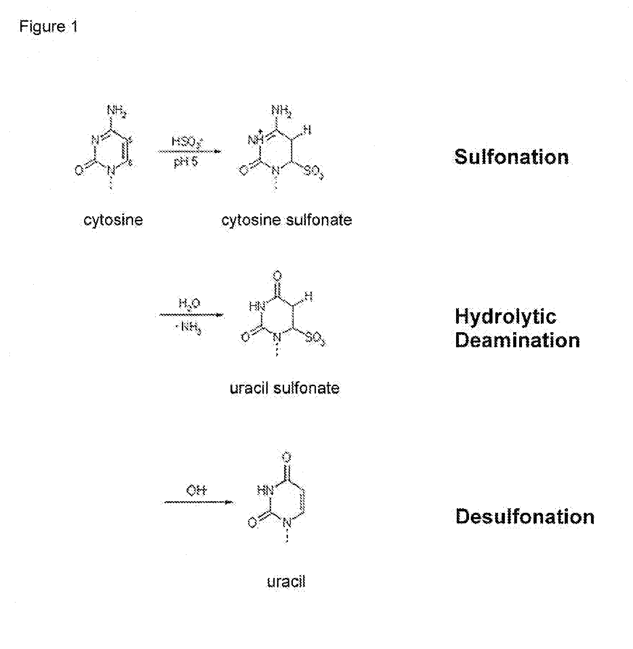

   Figure 1 shows the complete conversion of unmethylated cytosine into uracil (bisulfite conversion).
   In the first step of the reaction, unmethylated cytosine bases are reacted with hydrogen sulfite at a pH of approximately 5, and position C6 becomes sulfonated. In the second step, the deamination takes place spontaneously in an aqueous solution. As a result, cytosine sulfonate is converted into uracil sulfonate. The third step is the desulfonation, which takes place under alkaline conditions. In the process, uracil is produced.
Figure 2:
   Figure 2 shows a variation of the method according to invention, in which the primer that serves as a oligonucleotide, and the zymogen are arranged on different molecules, and through which the nucleic acid amplification as a "rolling circle" amplification (RCA) is carried out.
   In part A of Figure 2, a bisulfite-converted single-stranded DNA molecule (1) is shown, which is to be analyzed with regard to a particular methylation pattern. All of the unmethylated cytosine bases in the unconverted DNA to be examined are now converted to uracil. They are represented as a "u".
   The converted DNA molecule to be examined interacts with a linear ligatable single-stranded DNA molecule (2).
   This linear ligatable single-stranded DNA molecule (2) shows
   a methylation-specific or a gene- and methylation-specific ligation region (3),
   a first sequence (4) was chosen as an example, to which an oligonucleotide, which serves as a primer can bind in order to initiate a methylation-specific nucleic acid amplification, and
   a second sequence portion in the form of a zymogen, which encodes for a catalytic nucleic acid activity, for example, 10-23 DNAzyme.
   The ligation region (3) of the linear ligatable single-stranded DNA molecule comprises in this particular example at the positions, which correspond to the positions being examined of the ligatable single-stranded DNA molecules (1), guanine bases (highlighted). A complete hybridization with the sequence of the ligation region (3), i.e. without mismatches, is only possible in this example when these positions, in the original unconverted DNA molecules, were methylated and after the bisulfite conversion, are therefore present as cytosines and not as uracils. Of course it is also possible to provide a ligation region, which exhibits in one or both positions an "A" in order to obtain a signal when a position is unmethylated.
   Through the bisulfite conversion, the double-stranded DNA develops into two different single-stranded DNA molecules that are no longer complementary to each other. In order to detect the second strand after bisulfite conversion, a corresponding linear ligatable single-stranded DNA molecule is to be chosen. If a position to be examined is methylated, then the ligation region comprises correspondingly a cytosine; if the position is unmethylated, then the region comprises correspondingly a thymine, in order to achieve complete hybridization, in each case.
   At complete hybridization, a ring-like DNA molecule (minicircle) is formed through a ligase contained in a reaction mixture.
   In part B of Figure 2, the actual rolling-circle-amplification (RCA) is depicted. A primer, functioning as an oligonucleotide, initiates the amplification of the generated minicircle. Thereby, a molecule is amplified that represents a concatamer of the minicircle. It contains the catalytic nucleic acid activity (7) many times over. The catalytic nucleic acid activity is the reverse complementary sequence of the zymogen (5). The catalytic nucleic acid activity (7) is preferably a 10-23 DNAzyme.
   The substrate (8) binds to the catalytic nucleic acid activity (7) through hybridization. The catalytic nucleic acid activity (7) modifies thereupon the substrate (8) so that it can be detected. The substrate (8) is preferably an oligonucleotide, in particular a fluorescently labeled nucleic acid molecule in the form of a DNA-RNA chimera, which is labeled on one end with a fluorescent dye and on its other end with a quencher.
   The substrate (8) represented here exhibits at one end a fluorescent-dye and at the other end a quencher. Through the cleavage of the substrate (8), which is catalyzed by the catalytic nucleic acid activity (7), the fluorescent-dye and the quencher are separated from one another. As a result, the fluorescent signal from the dye can be detected.
   The product of the nucleic acid amplification shows with cumulative reaction time, an increasing number of active nucleic acid portions and with that increasing catalytic nucleic acid activity. If only one oligonucleotide is used for amplification, then the conversion of the substrate through the catalytic nucleic acid activity increases linearly with time. If, in contrast, two primers functioning as oligonucleotides are used, then an exponential increase in amplification of the catalytic nucleic acid activity is achieved. The conversion of the substrate is carried out correspondingly. At this juncture, an oligonucleotide binds specifically to a region of the minicircle and the other specifically to regions of the concatamer. In both cases, the detection of the signal allows one to draw conclusions on the existence of a methylation pattern in the DNA molecule being examined.
Figure 3:
   Figure 3 shows the 10-23 DNAzyme as a catalytic nucleic acid activity. The 10-23 DNAzyme has a catalytic domain and two flanking arms, which are each the reverse complementary sequences of the substrate represented by the upper strand. The sequence of both arms can be arbitrarily chosen. The cleavage site of the DNAzyme is identified with an arrow.
   In part A of the figure, an especially preferred variation of the 10-23 DNAzyme as a catalytic nucleic acid activity is presented.
   Part B of the figure shows a general model of the secondary structure of 10-23 DNAzyme where the different domains of 10-23 DNAzyme are summarized. A variety of nucleotides that make up the secondary structure are highly conserved. An N is used to represent a non-essential nucleotide where its replacement with another nucleotide would not result in a loss of over 80% of the catalytic activity. The region, which probably forms the catalytic domain, is marked with a dotted line. Exocyclic functional groups, which are necessary for the activity, are labeled.
   N': every other nucleotide that is complementary to N
   R: purine preference
   Y: pyrimidine preference
   R': complementary to Y or W
   M: A or C
   H:A,C,orT
   D: G, A or T
   V: ribonucleotide, either G, A or T
   W: C or G
Figure 4:
   Figure 4 shows the orientation of sequences of the Duplex real time PCR of example 1. Italicized letters represent the gene-specific sequences of the primer. Singly-underlined sequences are the zymogen region, which encodes the catalytic portion of the DNAzyme. Double-underlined sequences are the catalytic part of the respective DNAzyme. Upper case letters represent deoxyribonucleiotides, whereas lower case letters represent ribonucleiotides. The region of the amplificates depicted in bold represents the region to which the Reporter Substrate specifically binds. The abbreviation of the modifications mean: FAM = 6-Carboxyfluoresceine, HEX = 6-Hexachlorofluoresceine, BHQ1 = BlackHoleQuencher1. (The orientation of the sequences to one another is shown in Figure 4).

### Definitions

The term "methylation pattern" refers to, in particular, but is not limited to, the presence or absence of methylation of one or a number of nucleotides. The said one or more nucleotides may be located on a single DNA molecule and can be methylated or unmethylated. Even if only one nucleotide is considered, then the term "methylation-status" may be used. If the methylation pattern for more than one DNA molecule is considered, then one can consider using the terms "methylation level" or "methylation pattern level". The given terms "methylation status" and "methylation level" and "methylation pattern level" refer to, in particular, but are not limited to, the terms as stated.

The term "methylation-specific restriction enzyme" refers to, in particular, but is not limited to, enzymes, which cleave a nucleic acid sequence when the recognition site is either unmethylated, or hemi-methylated or cleaves only when the site is methylated. A restriction enzyme cuts specifically when the recognition site is either unmethylated or hemi-methylated and does not carry out the cleavage at all or with reduced efficiency if the recognition site is methylated. A restriction enzyme that cuts specifically when the recognition site is methylated does not carry out the cleavage at all or with reduced efficiency if the recognition site is unmethylated. Preferred are methylation-specific restriction enzymes, which have recognition sites that contain a CG-dinucleotide (for example, cgcg or cccggg). Furthermore preferred for some embodiments are restriction enzymes, which do not cleave when the cytosine in the dinucleotide is methylated at carbon 5 (C5). Each and every enzyme that fulfills this criterion is, according to the invention, applicable.

The term "zymogen" refers to, in particular, but is not limited to, a nucleic acid, which encodes a catalytic nucleic acid activity. This nucleic acid thereby comprises itself the reverse complementary sequence of the catalytic nucleic acid activity.

The term "catalytic nucleic acid activity" refers to, in particular, but is not limited to, a nucleic acid, which takes part in a chemical reaction without it being used up in the process. Such a nucleic acid accelerates a reaction by limiting the activation energy needed for the reaction.

The term "concatamer" (also concatemer) refers to, in particular, but is not limited to, a nucleic acid molecule that comprises repeating units.

The term "fluorophor" is used here synonymously with the term "fluourescent-dye". In this case, it concerns a substance that is capable of fluorescing. It absorbs energy from a particular wavelength or wavelength region and re-emits the energy in another wavelength or wavelength region.

## Claims

1. Method for detecting the presence of one or more methylation patterns, comprising
a) the conversion of the nucleic acid to be examined so that 5-methylcytosine remains unchanged, while unmethylated cytosine is changed into uracil or another base, which can be distinguished from cytosine in its base-paring behavior, and
b) a catalytic nucleic acid activity

2. Method of claim 1 for detecting the presence of a methylation pattern in the nucleic acid of a sample, comprising
a) the conversion of a sample nucleic acid to be examined so that 5-methylcytosine remains unchanged, while unmethylated cytosine is changed into uracil or another base, which can be distinguished from cytosine in its base-paring behavior,
b) the subjecting of the nucleic acid to be examined to
i) an oligonucleotide appropriate for the initialization of nucleic acid amplification and
ii) a zymogen, encoding a nucleic acid catalytic activity, which is its reverse complementary sequence,
under conditions that allows for a primer-initiated nucleic acid amplification and a catalytic activity of nucleic acid activity wherein the oligonucleotide and the zymogen are oriented with one another in the presence of the nucleic acid to be examined such that an amplificate is generated, which comprises either the same sequence as the nucleic acid sequence to be examined or the reverse complementary sequence, as well as, the catalytic nucleic acid activity, and
iii) the detection of the catalytic nucleic acid activity through which the presence of a methylation pattern in the nucleic acid to be examined is determined.

3. Method of claim 1 for detecting the presence of a methylation pattern in a mixture of different nucleic acids, comprising
a) the conversion of various nucleic acids to be examined, so that 5-methylcytosine remains unchanged, while unmethylated cytosine is changed into uracil or another base, which can be distinguished from cytosine in its base-paring behavior.
b) the subjecting of the converted nucleic acid to at least one oligonucleotide appropriate for initiating nucleic acid amplification,
c) the subjecting of a catalytic nucleic acid activity to the amplificates during nucleic acid amplification wherein a part of the amplificates is modified through the catalytic nucleic acid activity.
d) the detection of non-modified amplificates whereby the presence of a methylation pattern in the mixture of the different nucleic acid to be examined is determined.

4. Method of claims 1-3 further comprising the quantitative determination of the catalytic nucleic acid activity generated in step b) through which the incidence of a methylation pattern in the nucleic acid of a sample is quantified.

5. Method of claim 4 where the quantitative determination of the catalytic nucleic acid activity comprises
a) the comparison of the catalytic nucleic acid activity with a standard,
b) real-time quantification, or
c) the quantification of multiple catalytic nucleic acid activities relative to one another

6. Method of claims 1-3, wherein the oligonucleotide, which comprises a primer sequence, and the zymogen are arranged on the different molecules or the same molecule

7. Method of claim 6, wherein the nucleic acid amplification is a "rolling circle" amplification.

8. Method of claim 1 or 2, comprising the digestion of the DNA to be examined with one or multiple restriction enzymes.

9. Method of claim 8 comprising additionally the ligation of adaptors to the generated fragments.

10. Method of claim 8, further comprising the ligation of a zymogen to the generated fragment.

11. Method of claims 1-3, wherein the converted nucleic acid is brought into contact with at least two oligonucleotides, wherein each comprise a primer sequence, and wherein at least one of the oligonucleotides comprises the zymogen.

12. Method of claim 11, wherein the catalytic nucleic acid activity of an amplificate recognizes and cleaves the amplificate in *cis.*

13. Method of claim 1 or 2, wherein the nucleic acid to be examined is brought into contact with at least two oligonucleotides and wherein said oligonucleotides each comprise a primer sequence and a zymogen.

14. Method of at least one of the preceding claims, wherein the nucleic acid to be examined is genomic DNA, DNA or RNA.

15. Method of at least one of the preceding claims, wherein the catalytic nucleic acid activity is either a Ribozyme or DNAzyme.

16. Method of at least one of the preceding claims, wherein the catalytic nucleic acid activity comprises the detectable modification of at least one substrate.

17. Method of claim 16, wherein the detectable modification is chosen from the group comprising: the formation and cleavage of a phosphodiester bond; nucleic acid ligation and cleavage; porphyrin metallation; formation of a carbon-carbon, ester, and amide bond.

18. Method of either claim 16 or 17, wherein the detectable modification comprises the cleavage of a fluorescently labeled nucleic acid molecule.

19. Method of claim 18, wherein the fluorescently labeled nucleic acid molecule is a DNA-RNA chimera.

20. Method of at least one of the preceding claims, wherein at least one oligonucleotide comprises a region, which functions as a methylation-specific primer.

21. Method of at least one of the preceding claims, further comprising methylation-specific blocking molecules.

22. Method of at least one of the preceding claims, comprising an additional methylation-nonspecific- or a methylation-specific amplification of the converted DNA.

23. Method of claim 1 for detecting the presence of multiple methylation patterns in a nucleic acid sample, comprising
a) the conversion of various nucleic acids to be examined, so that 5-methylcytosine remains unchanged, while unmethylated cytosine is changed into uracil or another base, which can be distinguished from cytosine in its base-paring behavior,
b) the subjecting of the nucleic acid to be examined to
i) multiple oligonucleotides, wherein for each methylation pattern there is at least one oligonucleotide, which is appropriate for the initiation of nucleic acid amplification, and
ii) multiple zymogens, wherein for each methylation pattern there is at least one zymogen, which encodes a nucleic acid catalytic activity that is measurable and produces the reverse complementary sequence, under conditions, which allows for a primer-initiated nucleic acid amplification and an activity of the catalytic nucleic acid activity wherein the oligonucleotide correlating with a methylation pattern and the zymogen correlating with a methylation pattern are oriented to one another such that in the presence of the nucleic acid to be examined that a nucleic acid molecule is generated, which comprises a sequence or the reverse complementary sequence of the nucleic acid sequence to be examined, as well as, the catalytic nucleic acid activity, and
c) the simultaneous detection of each catalytic nucleic acid activity through which the presence of a methylation pattern in the nucleic acid to be examined is determined.

24. Method of claim 23 further comprising the quantitative determination of each catalytic nucleic acid activity generated in step b) through which the incidence of a methylation pattern of a nucleic acid in the sample is quantified.

25. Method of at least one of the preceding claims, wherein the conversion of nucleic acid comprises bisulfite.

26. Method of at least one of the preceding claims, wherein the nucleic acid amplification is carried out according to a method from the group comprising PCR, SDA, and TMA.

27. Method of at least one of the preceding claims, wherein the presence of a methylation pattern in a sample indicates a disease.

28. Oligonucleotide, comprising
a) a sequence portion, comprising a catalytic nucleic acid activity or a sequence, which encodes a catalytic nucleic acid activity, and
b) a sequence portion, which
i) comprises the bases A, C, T, or G, which are appropriately positioned for distinguishing between converted methylated and un-methylated positions in the nucleic acid to be examined; and
ii) all other positions comprise only the bases A, T, C, or only the bases A, T, G.

29. Oligonucleotide of claim 28, comprising additionally of one or more sequence portions, which either itself or the reverse complimentary sequence forms a bond with a substrate.

30. Kit for implementing the method of one of the preceding claims, comprising:
a) a reagent or enzyme, which makes a conversion of the nucleic acid to examined possible, such that 5-methylcytosine remains unchanged, while the unmethylated cytosine is converted into uracil or another base, which can be distinguished from cytosine in its base-paring behavior,
b) a catalytic nucleic acid activity or a zymogen, which encodes a catalytic nucleic acid activity.

31. Kit of claim 30, further comprising
a) an oligonucleotide appropriate for the initialization of nucleic acid amplification, and/or
b) a methylation-specific blocking molecule.

32. Kit of claim 30 or 31, further comprising a substrate that is detectably modified through a catalytic nucleic acid activity.

33. Kit of claims 31 and 32, wherein a region of the oligonucleotide, which enables the initiation of nucleic acids,
a) comprises the bases A, C, T, or G in positions, which are suitable to distinguish between converted methylated and un-methylated positions in the nucleic acid to be examined; and
b) all other positions comprise only the bases A, T, C, or only the bases A, T, G.
